(19) Europäisches Patentamt
European Patent Office
Office européen des brevets

(11) **EP 4 083 610 B1**

(12) **EUROPEAN PATENT SPECIFICATION**

(45) Date of publication and mention of the grant of the patent:
**17.09.2025 Bulletin 2025/38**

(21) Application number: **20906413.8**

(22) Date of filing: **04.11.2020**

(51) International Patent Classification (IPC):
**G01N 21/64** (2006.01) **B82Y 30/00** (2011.01)
**G01N 33/58** (2006.01) **G01N 21/77** (2006.01)
**G01N 33/553** (2006.01)

(52) Cooperative Patent Classification (CPC):
**G01N 21/645; G01N 33/553; G01N 33/582;**
B82Y 30/00; G01N 21/6428; G01N 2021/6482;
G01N 2021/7763; G01N 2021/7786;
G01N 2021/7789

(86) International application number:
**PCT/JP2020/041153**

(87) International publication number:
**WO 2021/131331 (01.07.2021 Gazette 2021/26)**

(54) **BIOMOLECULAR INSPECTION CHIP FOR FLUORESCENCE DETECTION**

BIOMOLEKULARER INSPEKTIONSCHIP FÜR FLUORESZENZDETEKTION

PUCE D'INSPECTION BIOMOLÉCULAIRE POUR DÉTECTION DE FLUORESCENCE

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **23.12.2019 JP 2019231043**

(43) Date of publication of application:
**02.11.2022 Bulletin 2022/44**

(73) Proprietor: **National Institute for Materials Science**
**Tsukuba-shi, Ibaraki 305-0047 (JP)**

(72) Inventor: **IWANAGA Masanobu**
**Tsukuba-shi, Ibaraki 305-0047 (JP)**

(74) Representative: **TBK**
**Bavariaring 4-6**
**80336 München (DE)**

(56) References cited:
WO-A1-2011/002010     WO-A1-2012/070175
WO-A1-2012/120874     JP-A- 2005 140 682
US-A1- 2006 098 927   US-A1- 2010 006 774
US-A1- 2010 330 578   US-A1- 2014 154 668

US-A1- 2014 206 101     US-A1- 2016 018 331
US-A1- 2019 302 022

- IWANAGA MASANOBU: "All-Dielectric Metasurfaces with High-Fluorescence-Enhancing Capability", APPLIED SCIENCES, vol. 8, no. 8, 9 August 2018 (2018-08-09), pages 1328, XP093104586, DOI: 10.3390/app8081328
- CHORSI HAMID T ET AL: "Patterned Plasmonic Surfaces-Theory, Fabrication, and Applications in Biosensing", JOURNAL OF MICROELECTROMECHANICAL SYSTEMS, IEEE SERVICE CENTER, US, vol. 26, no. 4, 1 August 2017 (2017-08-01), pages 718 - 739, XP011657920, ISSN: 1057-7157, [retrieved on 20170731], DOI: 10.1109/JMEMS.2017.2699864
- CHOI BONGSEOK ET AL: "Overcoming metal-induced fluorescence quenching on plasmo-photonic metasurfaces coated by a self-assembled monolayer", vol. 51, no. 57, 1 January 2015 (2015-01-01), UK, pages 11470 - 11473, XP093225179, ISSN: 1359-7345, Retrieved from the Internet <URL:https://pubs.rsc.org/en/content/articlepdf/2015/cc/c5cc04426j> [retrieved on 20241126], DOI: 10.1039/C5CC04426J

**EP 4 083 610 B1**

- **IWANAGA MASANOBU ET AL: "The artificial control of enhanced optical processes in fluorescent molecules on high-emittance metasurfaces", NANOSCALE, vol. 8, no. 21, 28 April 2016 (2016-04-28), United Kingdom, pages 11099 - 11107, XP093271416, ISSN: 2040-3364, Retrieved from the Internet <URL:https://pubs.rsc.org/en/content/articlepdf/2016/nr/c6nr01318j> [retrieved on 20241126], DOI: 10.1039/C6NR01318J**

**Description**

**Art Field**

The present invention relates to a biomolecular inspection chip configured for fluorescence detection.

**Background Art**

There is a biomolecular inspection method making use of surface plasmon resonance (SPR) (see Patent Publication 1 as an example).

A surface plasmon resonance sensor is a device designed to detect a surface plasmon resonance phenomenon occurring on a surface of metallic thin film such as gold or silver. The surface plasmon is a sort of compressional wave of electrons appearing on a metal-dielectric interface, with its wavenumber changing depending on the thickness (of up to several hundred nm) and optical characteristics (such as dielectric constant and refractive index) of a sample or specimen in contact with the surface of the metallic thin film. It is impossible to directly measure this change; in a typical method of using the SPR sensor, therefore, laser light is irradiated from the opposite surface of the sample for generation of evanescent waves such that they resonate with surface plasmon, and changes in the incident angle of laser are measured to indirectly detect changes in the surface state. Patent Publication 1 suggests that if the sensor surface is chemically modified with polymers having a variety of characteristics and functions, it is then possible to provide a sensor device capable of detecting more biological components than ever. Specifically, it states that if a metallic thin film is used as a sensor substrate and an SPR sensor is used as a device, it is then possible to provide an immunosensor making use of antigen-antibody reactions, showing the result of detection of 0.1 μg/ml of antibody immunoglobulin G at the lowest concentration. Now that about 5 ng/ml are set as the basic value in disease diagnosis, however, Patent Publication 1 would fail to provide any accurate enough method.

Among methods used so far for medical diagnosis or inspection there is ELISA method in which enzymatic reactions are introduced in biomolecules of interest thereby verifying the concentration of the target molecule from the absorbance of a sample. This method has now been established as a standard, enough accurate method. Immunoglobulin G (IgG) that is a representative antibody having a double-chain, double-light chain structure common to many antibodies ensures a detection of about 15 ng/ml. Although biomolecular inspection with accuracy of higher than 1 ng/ml is needed for diagnosis of early-stage cancers or the like, any method meeting such demand is not available so far.

Fluorescence inspection is known for biomolecular inspection (see Patent Publications 2 and 3 as an example). In this fluorescence inspection, a target substance excited by excitation light to generate fluorescence or a fluorescently labeled target substance is irradiated with excitation light to detect fluorescence thereby detecting the target substance.

Patent Publication 2 discloses a microchannel chip comprising a reaction chamber, an inlet and an outlet, a supply channel and a discharge channel adapted to connect the reaction chamber to the inlet and the outlet, respectively, characterized by including a recessed substrate holder, a reaction substrate located in the recess of the substrate holder, a first sheet disposed in such a way as to cover the substrate holder and the reaction substrate, and a second sheet disposed in such a way as cover the first sheet, wherein the first sheet has a through hole by which the reaction chamber is defined between the second sheet and the reaction substrate, the reaction substrate has a first surface exposed to the reaction chamber and a second surface exposed out via an observation window located in the recess of the substrate holder, and the first surface of the reaction substrate is provided with a reaction spot comprising a microstructure formed to generate a localized type surface plasmon.

While Patent Publication 2 states that the reaction spot is combined with a fluorescently labeled single primer molecule to capture a fluorescently labeled dNTP molecule, and discloses a configuration wherein these fluorescent dyes are excited by prismatic evanescent light and emit light to observe localized light emission using the localized type surface plasmon, it discloses nothing about the effects of detection (such as limit of detection and detection sensitivity).

In a biochip module according to the prior art such as Patent Publication 3, on the other hand, the substance of interest introduced into a laser cavity region held between mirrors is irradiated with excitation light, and the excited fluorescence is resonated in a thickness direction of the laser cavity for light intensity enhancement. Even when such laser cavity resonance is used, however, sufficient fluorescence intensity would not be obtained.

In recent years, artificial nanostructure surfaces (also called metasurfaces) have been studied all over the world, inclusive of a metasurface having excellent fluorescence enhancing capability (see Patent Publication 4 and Non-Patent Publications 1, 2 and 3 as examples). Patent Publication 4, and Non-Patent Publication 3 discloses a substrate for surface reinforcing Raman analysis comprising a substrate material, a slab material positioned on a surface of the substrate, and a metallic material positioned on at least the slab material and being capable of enhancing an optical response of the substance to be tested. The substrate material includes a surface layer coming in contact with at least the slab material, and the slab material comprises a material having a refractive index higher than that of the surface layer and includes a plurality of periodically arranged holes extending from the surface of the slab material to the surface layer of the substrate material, and the metallic material is positioned on the surface of the slab material and the surface layer of the substrate material via the plurality of respective holes, includes a complementary metal structure and comprises a plurality of resonances determined by the diameters and periods of the plurality of holes.

Referring further to Non-Patent Publication 1, an SOI (silicon on insulator) substrate is provided with a plurality of silicon nanorods by means of electron beam lithography (EBL) and BOSCH process, thereby allowing this substrate to have significant fluorescence enhancement capability. In addition, Non-Patent Publication 1 suggests a possible application of such a substrate having a plurality of such nanorods to detection of fluorescence from biomolecules.

Non-Patent Publication 2 reports that if a self-assembled monolayer (SAM) is imparted on the complementary metal structure shown in Patent Publication 1, a rhodamine dye fluorescent signal increases several ten times as large as a case with no SAM.

When the metasurfaces shown in Patent Publication 4 and Non-Patent Publications 1 to 3 are applied to a fluorescence detection method, however, biomolecules in a liquid are not efficiently immobilized to metasurface alone; hence, it is impossible to provide high-precision fluorescence detection method needed for diagnosis of diseases, and so on.

### Prior Arts

#### Patent Publications

    Patent Publication 1: Patent No. 3657790
Patent Publication 2: JP(A)2011-220996
Patent Publication 3: JP(A)2006-177878
Patent Publication 4: JP(A)2017-173084

#### Non-Patent Publications

    Non-Patent Publication 1: Masanobu Iwanaga, Appl. Sci., 2018, 8, 1328
Non-Patent Publication 2: Bongseok Choi, et al., Chem. Commun. 2015, 51, 11470-11473
Non-Patent Publication 3: Masanobu Iwanaga, et al., Nanoscale, 2016, 8, 11099-11107

Further prior art documents are WO 2011/002010 A1 showing a high-sensitivity fluorescence detection device and US 2014/206101 A1 showing a plasmon resonance imaging apparatus having nano-lycurgus-cup arrays and methods of use.

### Summary of the Invention

#### Problems with the Prior Art

As can be seen from the foregoing, the present invention has for its object to provide an inspection chip using a metasurface for the purpose of detecting biomolecules by the fluorescence detection method.

#### Objects of the Invention

According to one embodiment the invention is directed to a biomolecular inspection chip for fluorescence detection as defined in claim 1. According to a further embodiment the invention is directed to a biomolecular inspection chip for fluorescence detection as defined in claim 6.

In one embodiment, the fluorescence enhancement may be intensity enhancement of light having a wavelength in a visible light region or a near infrared light region.

In one embodiment, the metallic material may be a substance having a complex dielectric constant approximate to that of a Drude metal.

The substance having a complex dielectric constant approximate to that of a Drude metal may be selected from the group consisting of gold (Au), silver (Ag), copper (Cu), aluminum (Al), platinum (Pt), titanium (Ti), nickel (Ni), and alloys of these metals.

In one embodiment, the plurality of holes may comprise round holes having two or more different diameters or prismatic holes having two or more one-side lengths, and/or may be arranged in two or more different periods.

In one embodiment, the plurality of holes may have a period of 300 nm to 1000 nm inclusive.

In one embodiment, the plurality of holes may have a diameter or one-side length of 100 nm to 500 nm inclusive.

In one embodiment, the slab material may have a thickness of 100 nm to 2 $\mu$m inclusive.

In one embodiment, the substrate material may comprise a material having a refractive index of 1 to 1.6 inclusive, and the plurality of nanorods may comprise a semiconductor or a dielectric material having a refractive index of 2 to 5 inclusive.

In one embodiment, the semiconductor or dielectric material may be selected from the group consisting of silicon, germanium, gallium nitride, and titanium dioxide.

The plurality of nanorods may have a period of 300 nm to 1000 nm inclusive.

In one embodiment, the plurality of nanorods may have a diameter or one-side length of 100 nm to 500 nm inclusive.

In one embodiment, the plurality of nanorods may have a height of 100 nm to 2 $\mu$m inclusive.

In one embodiment, the plurality of nanorods may comprise round columns having two or more different diameters or prismatic columns having two or more different one-side lengths, and/or may be arranged in two or more different periods.

## Advantages of the Invention

According to the biomolecular inspection chip for fluorescence detection of the invention, the gap for efficient capture and immobilization of a biomolecule is provided, and the first substrate having significant fluorescence enhancement capability is positioned in opposition to the second substrate comprising a microchannel so that fluorescence can be detected from even a biomolecule in a liquid sample. In the inspection chip of the invention, the first and second substrates and a $\mu$m-order space between both substrates define together a microresonator that allows for resonance of fluorescence occurring from the biomolecule. In that case, the metasurface of the first substrate serves to enhance fluorescence simultaneously with efficient radiation of fluorescence toward the second substrate side; hence, radiation of fluorescence from the microresonator is put together in one direction, resulting in more efficient fluorescence detection. As a result, even given bio-molecules in lower concentrations, it is possible to detect fluorescence with higher sensitivity and reproducibility than ever before.

## Brief Explanation of the Drawings

Fig. 1 is a schematic view of the biomolecular inspection chip for fluorescence detection according to the invention.

Fig. 2 is a schematic view of the detection principles on which the biomolecular inspection chip for fluorescence detection according to the invention is used.

Fig. 3 is a schematic view of the metasurface having a metal complementary laminated structure.

Fig. 4 is a schematic view of the metasurface having a nanorod structure.

Fig. 5 is a schematic view of a detection apparatus assembled with the inspection chip according to the invention.

Fig. 6 is a flow diagram of how the biomolecular inspection chip for fluorescence detection according to the invention is used to determine whether or not a target biomolecule is present in a sample solution.

Fig. 7 is an SEM image indicative of the metasurface having a silicon nanorod structure.

Fig. 8 is an SEM image indicative of the metasurface comprising a gold complementary laminated structure.

Fig. 9 illustrates an external appearance of the inspection chip of Example 1 (A), and an external appearance of the flow channel device incorporating it (B) .

Fig. 10 illustrates an external appearance of the inspection chip according to Example 3.

Fig. 11 is illustrative of a post-inspection state of the inspection chip of Example 1.

Fig. 12 is indicative of emission spectra obtained by using the inspection chip of Example 1.

Fig. 13 is indicative of emission spectra obtained by using the inspection chip of Comparative Example 1.

Fig. 14 is indicative of a standard curve obtained through fluorescence measurement according to Example 1.

Fig. 15 is indicative of a standard curve obtained through fluorescence measurement according to Example 2.

Fig. 16 is indicative of a standard curve obtained through fluorescence measurement according to Example 3.

Fig. 17 is indicative of a standard curve obtained through fluorescence measurement according to Example 4.

Fig. 18 is indicative of a standard curve obtained through fluorescence measurement according to Example 5.

Fig. 19 is indicative of a standard curve obtained through fluorescence measurement according to Comparative Example 1.

Fig. 20 is indicative of a standard curve obtained through fluorescence measurement according to Comparative Example 2.

Fig. 21 is indicative of fluorescence intensities compared in terms of the presence or absence of captured antibodies according to Example 6.

Fig. 22 is a fluorescent image of the inspection chip according to Example 8.

Fig. 23 shows comparisons of fluorescence intensities among various buffers used in Example 9.

Fig. 24 shows fluorescent images of the respective channels in Example 10 and schematic views of each state where biomolecules are immobilized in place.

Fig. 25 is a diagram indicative of comparisons of fluorescence intensities in Example 10.

Fig. 26 is a schematic view of a molecular appearance on the metasurface of the inspection chip in Example 11.

Fig. 27 is a set of views showing CEA fluorescent images according to Example 11 in various concentrations.

Fig. 28 is a diagram indicating of concentration dependency of CEA fluorescence intensity obtained from fluorescence measurement in Example 11.

## Modes for Carrying Out the Invention

Embodiments of the present invention will now be explained with reference to the accompanying drawings. It is here noted that like numerals are given to like elements; they will not be explained anymore.

The biomolecular inspection chip for fluorescence detection according to the invention is first explained.

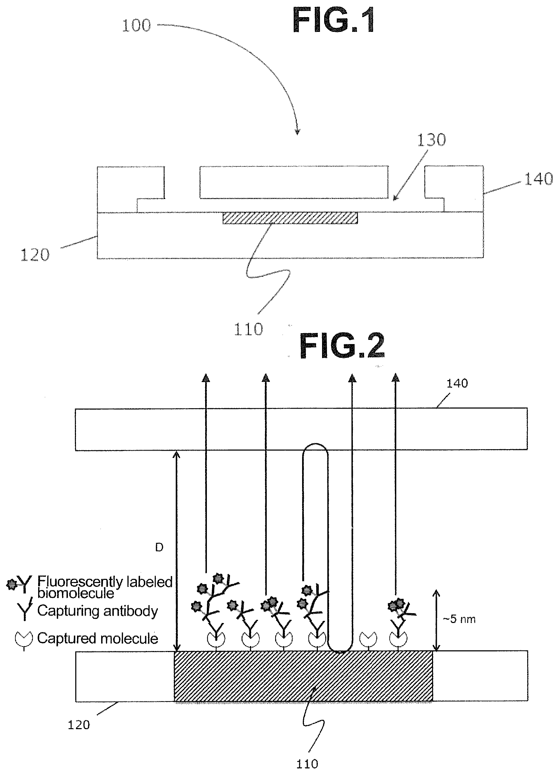

Fig. 1 is a schematic view of the biomolecular inspection chip for fluorescence detection according to the invention, and Fig. 2 is a schematic view of the detection principles on which the biomolecular inspection chip for fluorescence detection according to the invention is used.

A biomolecular inspection chip 100 for fluorescence detection according to the invention comprises a first substrate 120 including a metasurface 110, and a second substrate 140 that is positioned in opposition to the metasurface 110 side and includes a microchannel 130. The metasurface 110 includes a gap for efficient immobilization of the biomolecule to be detected (hereafter called the target biomolecule for simplicity), and serves well to enhance fluorescence.

It is here understood that the "target biomolecule" refers to biomolecules such as antibodies, antigens, peptide of III type procollagen and by-product proteins, inclusive of Hepatitis virus IgM type antibody, Hepatitis virus s antigen, CEA molecule, p53 molecule and p53 antibody as well as nucleic acids such as RNA and DNA, and subdivided molecules thereof.

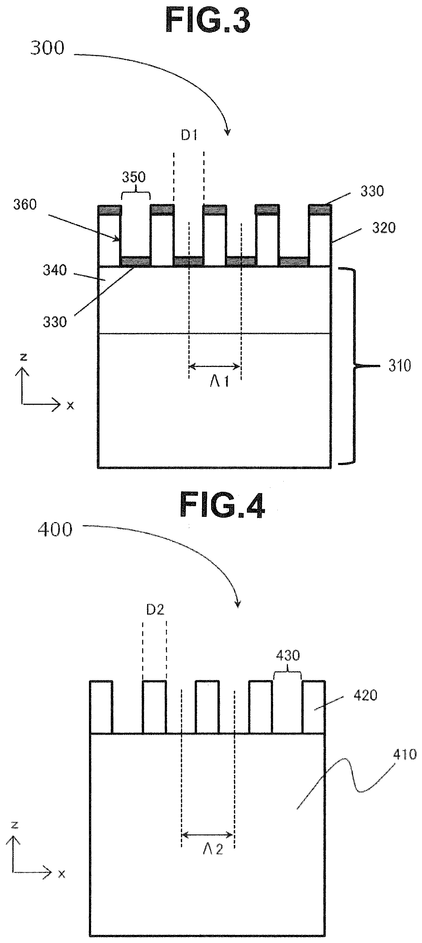

Intended by the "metasurface including gaps" is a surface comprising a stereo-structure with gaps that is larger than a biomolecule and of the order of several tens to several hundred nanometers. By including such gaps, the metasurface 110 is allowed to have an increased surface area in such a way as to make immobilization of bio-molecules efficient. This also serves to reduce a fluid flow rate locally, again leading to efficient immobilization of biomolecules. Although both Figs. 1 and 2 do not show the gaps because the former is a macroscopic view and the latter illustrates biomolecules on an enlarged scale, the metasurface 110 includes gaps. Fig. 2 provides an illustration of a state where a capturing molecule is immobilized on the metasurface 110, a capturing antibody is bound to the captured molecule, and a fluorescently labeled biomolecule is efficiently bound to the antibody. As such, the metasurface 110 can make immobilization of biomolecules efficient. The gap is indicated by numeral 350 in Fig. 3 to be described later, and 430 in Fig. 4 to be described later.

Intended by the "metasurface inducing fluorescence enhancement" is an artificial nanosurface structure as described above, wherein as a fluorescence-emitting material is positioned thereon, it causes fluorescence intensity to be greater as compared with when it is placed on a flat surface such as a silicon wafer or a highly smoothed quartz substrate. According to the inspection chip 100 of the invention, the metasurface 110 of the first substrate 120 further serves to enhance fluorescence in a region including a wavelength range of fluorescence emitted by the target biomolecule (for instance, the fluorescently labeled biomolecules in Fig. 2) so that even when the concentration of the target biomolecule is low, that concentration can be detected with high accuracy. Here note that intended by the "fluorescence emitted by the target biomolecule" is fluorescence emitted by the target biomolecule itself, fluorescence emitted by a fluorescent label applied on the target biomolecule or fluorescence emitted by a fluorescent label applied on a secondary antibody captured by the target biomolecule.

The second substrate 140 in the inspection chip 100 of the invention comprises a material transparent to visible light or near infrared light (light-transmitting material). As the target biomolecules positioned on the first substrate 120 is irradiated with light (excitation light), it causes the target biomolecule or the fluorescent label applied thereon to be excited, emitting out fluorescence. The fluorescence emitted by the target biomolecule resonates between the first substrate 120 and the second substrate 140, as shown in Fig. 2. This results in enhancement of the intensity of fluorescence from the target biomolecule so that even when the concentration of the target biomolecule is low, that concentration can be detected with high accuracy. Further, the fluorescence enhanced by resonance is radiated toward the second substrate 140 side rather the metasurface 110 so that it can be efficiently detected through the second substrate 140 if it comprises a light-transmitting material. It is herein understood that intended by visible light is light having a wavelength range of 360 nm to less than 830 nm, and intended by near infrared light is light having a wavelength range of 830 nm to 1600 nm inclusive.

In the present disclosure, it is understood that the material transparent to visible light or near infrared light means a material having an average transmittance of 60% or higher relative to visible light or near infrared light, and more preferably of 80% or higher. For such a material transparent to visible light or near infrared light, inorganic materials such as transparent ceramics and glasses and organic materials such as plastics may be used; in terms of processability, however, the inspection chip of the invention uses silicone-based resin, (meth)acrylic-based resin, epoxy-based resin, styrene-based resin, polycarbonate, ester-based resin, acrylonitrile-butadiene-styrene resin, polyamide, cycloolefin polymer, among which polydimethylsiloxane (PDMS) that is a silicone-based resin is preferred.

The fluorescence enhancement induced or developed by the metasurface 110 refers to an enhancement in the intensity of light having a wavelength in the visible or near infrared region, preferably an enhancement in the intensity of light having a peak in a wavelength range of 520 nm to 1500 nm inclusive, resulting in an improvement in the detection precision of biomolecules. More preferably, this metasurface enhances light having a peak in a wavelength range of 540 nm to 620 nm inclusive or 800 nm to 900 nm inclusive.

The height of the microchannel formed by the first substrate 120 and the second substrate 140, that is, a distance D (Fig. 2) between the first substrate 120 and the microchannel through the second substrate 140 is according to the invention in a range of 15 $\mu$m to 50 $\mu$m inclusive, wherein the inspection chip could function evidently as a microresonator with an increase in efficiency with which the microchannel immobilizes the target biomolecule on the first substrate 120. When the distance D is shorter than 10 $\mu$m, meanwhile, it becomes difficult to provide any stable fluid flow within the channel or otherwise the second substrate 140 may deform due to its own weight, possibly leading to an undesired event such as contact with the first substrate 120.

Fig. 3 is a schematic view of the metasurface having a metal complementary stacked structure.

A metasurface 300 having a metal complementary stacked structure includes a substrate material 310, a slab material 320 positioned on a surface of the substrate material 310, and a metallic material 330 positioned on the slab material 320 and a surface layer 340 of the substrate material 310.

The substrate material 310 comprises the surface layer 340 that is in contact with at least the slab material 320 that is formed of a material having a refractive index higher than that of the surface layer 340. Further, the slab material 320 includes a plurality of periodically arranged holes 350 extending from the surface of the slab material 320 down to the surface layer 340 of the substrate material 310. Such a structure allows for the substrate material 310 and slab material 320 to have a resonance band state of light characteristic of a periodic structure determined by a period $\Lambda_1$ and a diameter $D_1$ of the plurality of holes 350 periodically arranged in a hexagonal, tetragonal, or other lattice configuration. In this case, the refractive index of the slab material 320 must be greater than that of the surface layer 340 so as to increase the band state density of light confined in the slab material 320 (confinement effect).

The metallic material 330 is positioned on the surface of the slab material 320 and the surface layer 340 of the substrate material 310 defining the bottoms of the plurality of holes 350, respectively, thereby creating a complementary metal stacked structure. In other words, the metallic material 330 does not cover the side walls of the plurality of holes 350 through the slab material 320, and the metallic materials 330 are typically spaced away from each other by just a distance obtained from subtracting the thickness of the metallic material 330 from that of the slab material 320, ensuring that the metasurface 300 having a metal complementary stacked structure can create a resonance state in which fluorescence emitted by the target biomolecule is confined within the slab material 320 through the holes' side walls 360 uncovered with the metallic material 330. Further, the incorporation of the metallic material 330 allows for the aforesaid resonance line widths to have a broadband so that at least one of plural such resonances overlaps with fluorescence coming from the target biomolecule, developing efficient fluorescence enhancement in its resonance line width range.

The slab material 320 is preferably formed of a material having a refractive index of 2 or more because, in most cases, materials having a refractive index of about 1.5 are used with the surface layer 340. Although there is no upper limit provided to the refractive index of the slab material 320, it has generally a refractive index of 5 or less in consideration of availability. Specifically, the material forming the slab material 320 may be selected from the group consisting of Si, Ge, SiN, SiC, II-VI compound semiconductors, III-V compound semiconductors and titanium dioxide ($TiO_2$). These materials, because of having a refractive index of 2 or more, could easily provide the slab material 320 through improved processability or by way of well-developed growth technologies.

The slab material 320 has preferably a thickness in a range of 100 nm to 2 $\mu$m inclusive, in which a band state of light is easily formed. If the slab material 320 having such a thickness range is combined with the metallic material 330 to form a complementary stacked structure, there is then a resonance state developed with an increased light emissivity, resulting in increased radiation efficiency of fluorescence emitted by the target biomolecule. More preferably, the slab material 320 has a thickness in a range of 150 nm to 250 nm inclusive, thereby enhancing the confinement effect of light in the slab material 320 and making it easier to allow for the complementary stacked structure combined with the metal material 330 to create a resonance state suitable for fluorescence enhancement (for instance, see Non-Patent Publications 2 and 3).

The plurality of holes 350 may have a period on the order of light wavelengths, which period is preferably in a range of 300 nm to 1000 nm inclusive. Within this range, the metasurface 300 can enhance light in a wavelength range of 520 nm to 1500 nm inclusive. The period $\Lambda_1$ is more preferably in a wavelength range of 400 nm to 750 nm inclusive. Within this range, the metasurface 300 can enhance light in a wavelength range of 540 nm to 900 nm inclusive. The plurality of holes 350 may be defined by those having two or more different periods. In this case too, if two or more different periods are in a range of 300 nm to 1000 nm, respectively, it is then possible to enhance light having a plurality of wavelength ranges in the visible light or near infrared light region so that it is also possible to detect two or more species of biomolecules having different fluorescent labels.

If the diameter $D_1$ of the plurality of holes 350 is smaller than the period $\Lambda_1$ and in a rage of 100 nm to 500 nm inclusive, the metasurface 300 can enhance light in a wavelength range of 540 nm to 1000 nm inclusive. The diameter $D_1$ is more preferably in a range of 250 nm to 350 nm inclusive, in which light in a wavelength range of 540 nm to 900 nm inclusive can be enhanced by the metasurface 300.

It is noted that the plurality of holes 350 may be defined by holes having two or more different diameters. In this case too, if two or more different diameters are in a range of 100 nm to 500 nm inclusive, respectively, it is then possible to enhance light in plural wavelength ranges in the visible or near infrared light region so that it is also possible to detect two or more species of biomolecules having different fluorescent labels.

As a matter of course, the plurality of holes 350 may be formed with two or more different diameters and arranged in two or more different periods, thereby gaining control of wavelength regions of visible light and near infrared light capable of fluorescence enhancement and, hence, addressing a variety of bio-molecules.

Although there is a cylindrical hole (round hole) shown in Fig. 3 by way of example alone, the holes may take other prismatic forms inclusive of triangular or quadrangular column forms; there is no limitation whatsoever provided that a given resonance state is created.

The surface layer 340 of the substrate material 310 is preferably formed of a material whose refractive index is less than 2. Although any limitation is not placed on the lower limit to the refractive index of the surface layer 340, the surface layer has a refractive index of generally 1 or higher in view of material availability. For the surface layer 340, there may be a so-called transparent insulator used inclusive of a material selected from the group consisting of $SiO_2$, $Al_2O_3$, glasses, and plastics that include a resin exemplified as the material of the second substrate 140. With these materials, light may be efficiently confined in the aforesaid slab material depending on the magnitude of refractive index. Note here that the substrate material 310 may be formed of a bulk substrate such as a Si or quartz substrate and the surface layer 340, and the bulk substrate could be defined by any desired substrate capable of holding the slab material 320 and metallic material 330 in place. For a metasurface material that is easily available and excels in processability, there is the mention of a material using a Si substrate for the substrate material 310 and $SiO_2$ for the surface layer 340, fused to the slab material 320 comprising Si. It is also possible to form the substrate material 310 by using a glass substrate or the like for the substrate material and depositing a Si layer thereon to provide the slab material 320.

The surface layer 340 has preferably a thickness equivalent to or greater than that of the slab material 320 whereby the slab material 320 functions as a waveguide of low light loss. More preferably, the surface layer 340 has a thickness of at least 200 nm.

Although there is no particular limitation on the metallic material 330, it has preferably a complex permittivity approximate to that of a Drude metal that means a metal obtained by modeling a metal having free electrons and represented by complex permittivity: $\varepsilon(\omega)=1-\omega_p^2/\omega(\omega+i\gamma)$ where $\omega$ is an angular frequency, $\omega_p$ is a plasma frequency, i is the imaginary unit, and $\gamma$ is a damping constant. It is generally known that many metals may be approximated as Drude metals except for the vicinity of a wavelength where there is electron band-to-band transition taking place (for instance, Frederich Wooten, Optical Properties of Solids, Academic Press, 1972, pp. 52-65). In the present disclosure, it is presumed that when the complex permittivity $\varepsilon(\omega)$ actually measured with $\omega_p$ and $\gamma$ as fitting parameters can be fitted to the aforesaid formula with a deviation of 20% at most, the metal material 330 of interest can be approximated by the Drude metal.

A substance having a complex permittivity approximate to that of such a Drude metal in the visible or near infrared light region is exemplified by a substance selected from the group consisting of gold (Au), silver (Ag), copper (Cu), aluminum (Al), platinum (Pt), titanium (Ti), nickel (Ni), and alloys thereof. The metallic material 330 has preferably a thickness of 30 nm to 100 nm inclusive. In a thickness of less than 30 nm, light will transmit through, often resulting in a loss of metal function. In a thickness of more than 100 nm, the side walls of each hole 350 will be closed up, often resulting in a failure in the creation of any complementary metal stacked structure. More preferably, the metal material 330 has a thickness in a range of 30 nm to 40 nm inclusive.

Fig. 4 is a schematic view of the metasurface having a nanorod structure.

A metasurface 400 having a nanorod structure comprises a plurality of upright nanorods 420 periodically provided on the surface of the substrate material 410. It is here noted that the substrate material 410 is preferably formed of a material having a refractive index lower than that of the nanorod 420 material.

a nanorod structure.

A metasurface 400 having a nanorod structure comprises a plurality of upright nanorods 420 periodically provided on the surface of the substrate material 410. It is here noted that the substrate material 410 is formed of a material having a refractive index lower than that of the nanorod 420 material.

When the substrate material 410 is formed of a material that is transparent in the wavelength region of light incident on the inspection chip 100 and has a refractive index of 1 to 1.6 inclusive, the nanorod 420 material is preferably a semiconductor or dielectric material having a refractive index of 2 to 5 inclusive, because the higher the refractive index of the nanorod material, the more prominent resonance state the nanorod develops. This more prominent resonance state is the physical condition needed to have resonance enhancement effects such as fluorescence enhancement effect, and can be easily identified by reflection or transmission spectra. As the substrate material 410 has a refractive index equal to or higher than that of the nanorod 420 material, on the other hand, it causes the resonance state to remain unclear with the result that any significant resonance enhancement effect will not be expectable.

300 nm to 1000 nm inclusive, the metasurface 400 can enhance light within a wavelength range of 520 nm to 1500 nm inclusive. The period $\Lambda_2$ has more preferably a range of 300 nm to 450 nm inclusive, allowing for the metasurface 400 to enhance light within a wavelength range of 540 nm to 900 nm inclusive. The plurality of nanorods 420 may have two or more different periods. Again, as is the case with the aforesaid metal complementary stacked structure, light having a plurality of wavelength ranges in the visible and near infrared light regions can be enhanced if the two or more different periods are each in a range of 300 nm to 1000 nm inclusive, also making two or more species of biomolecules having different fluorescent labels detectable.

As the plurality of nanorods having a diameter $D_2$ that is smaller than the aforesaid period $\Lambda_2$ and within a range of 100 nm to 500 nm inclusive, it allows for light in a wavelength range of 520 nm to 1500 nm inclusive to be enhanced by the metasurface 400 as is the case with said period $\Lambda_2$ in a preferable range. The diameter $D_2$ is more preferably within a range of 200 nm to 350 nm inclusive, thereby allowing for light within a wavelength range of 540 nm to 900 nm inclusive to be enhanced by the metasurface 400.

It is here understood that the plurality of nanorods 420 may have two or more different diameters. Again, as the two or more different diameters are each in a range of 100 nm to 500 nm inclusive, it allows for light in a plurality of wavelength ranges to be enhanced in the visible and near infrared light regions, making it possible to detect two or more species of biomolecules having different fluorescent labels.

As a matter of course, the plurality of nanorods 420 may be arranged or arrayed in two or more different periods and with two or more different diameters, thereby gaining accurate control of the wavelength region of light capable of fluorescence enhancement and addressing a variety of biomolecules.

The plurality of nanorods 420 has preferably a height in a range of 100 nm to 2 $\mu$m inclusive, in which an effect of electromagnetic resonance localized in the nanorods becomes so noticeable that there can be a prominent resonance state developed. More preferably, the plurality of nanorods 420 have a height in a range of 150 nm to 250 nm, thereby enabling to make use of low-order resonance states so that much more fluorescence enhancement could be expected.

Although each nanorod is designed to take a cylindrical form shown in Fig. 4 by way of example alone, it may take other prismatic column forms inclusive of triangular or quadrangular column forms; there is no limitation whatsoever provided that a given resonance state is created.

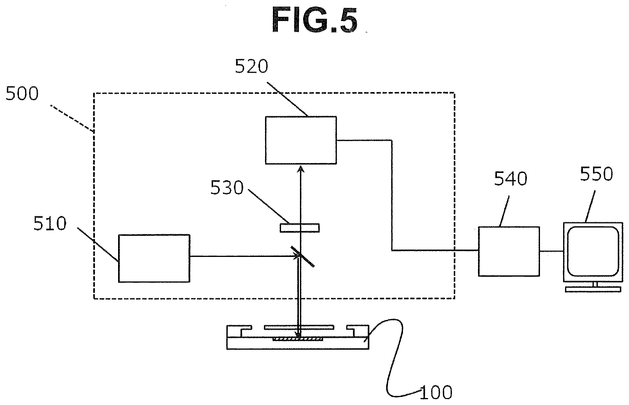

Fig. 5 is a schematic view of the inspection apparatus using the inspection chip according to the present invention.

Inspection apparatus 500 comprises at least a light source 510 configured to irradiate an inspection chip 100 with excitation light, and a detection means 520 configured to detect fluorescence radiated from the inspection chip 100.

The light source 510 used herein includes a xenon lamp, a light-emitting diode, a laser diode, a semiconductor laser, an organic EL light emitter, or the like. The inspection apparatus 500 may further include optical systems such as a wavelength selection filter, a mirror, a beam splitter such that excitation light from the light source 510 is incident on the inspection chip 100 in the desired wavelength at the desired angle to irradiate the metasurface 110 through the second substrate 140 shown in Fig. 1.

The detection means 520 used herein may be provided by any desired detection means provided that they are capable of detecting fluorescence excited by excitation light, typically inclusive of a photodetector, a CCD camera, a CMOS camera, a photodiode array, and a spectrometer, and the detection apparatus 500 may further include optical systems such as an objective lens and an image formation lens.

Further, the detection apparatus 500 may include a filter 530 configured to separate excitation light and fluorescence so as to hold down stray light, making accurate light measurement possible with low backgrounds.

In addition, the detection apparatus 500 may incorporate a computer 540 adapted to acquire data from the detection means 520 and analyze the data. Based on the received data, the computer 540 may analyze signals, backgrounds, and so on. For example, when the computer 540 comprises memories having data stored therein about relationships between fluorescence intensity and concentration, between fluorescent labels and their emission peaks, between fluorescent labels and various biomolecules, and so on, it is then possible to identify the species of biomolecules detected out of the acquired data, calculate the concentration of the identified biomolecule, and so on. Further, data analyzed by the computer 540 may be outputted and displayed on an output apparatus 550 such as a display, a printer or the like.

The detection apparatus 500 may further comprise feed containers adapted to hold one or more sample solutions, reagents for capturing molecules, capturing antibodies, etc., and washing liquids, and recovery containers adapted to hold post-measurement wastes as well as dispenser mechanisms (not shown) connected to these containers. Operation of the dispenser mechanisms may be controlled by the computer 540 thereby rendering automatic detection/analysis possible.

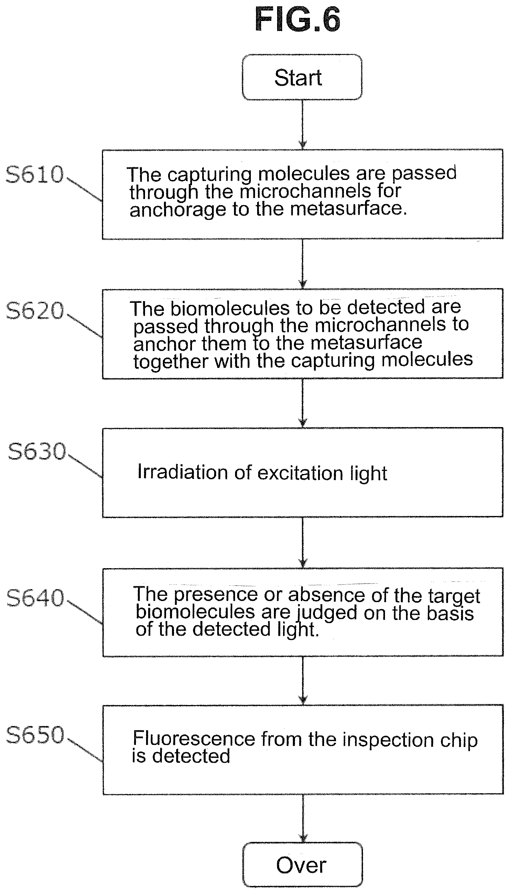

Fig. 6 is a flow diagram for making determination of whether or not the target biomolecules are present in a sample solution using the biomolecular inspection chip for fluorescence detection according to the present invention.

One exemplary process of determining the presence or absence of the target biomolecule with the detection apparatus 500 of Fig. 5 will now be explained in detail for each step.

In Step S610, a solution containing capturing molecules for the purpose of capturing the target biomolecule is passed through a microchannel 130 of an inspection chip 100 depicted in Fig. 1, followed by immobilization on a metasurface 110. Because the metasurface 110 includes a gap, passage of the solution of the capturing molecules through the microchannel 130 is all that is needed for easy immobilization of the molecules. Such capturing molecules may optionally be selected depending on the target biomolecule. Prior to passage of the solution containing the capturing molecules, a buffer such as a phosphate-buffered saline (PBS) may be passed through the microchannel to keep it full of liquid.

For instance, when the target biomolecule is a protein such as immunoglobulin, any capturing molecule capable of capturing the protein by way of FC binding may be used. A typical example of such a capturing molecule is an antibody binding protein or an avidin derivative.

The antibody binding protein is selected from the group consisting of protein A, protein G, protein AG, and derivatives thereof. Although there is no particular limitation on the avidin derivative capable of binding to biotin, it is preferable to select the avidin derivative from the group consisting of streptavidin, avidin, neutravidin, and derivatives thereof.

The target biomolecule may be a nucleic acid such as RNA or DNA other than the protein. In this case, a complementary DNA (normally called Aptamer) capable of specifically capturing the nucleic acid by hybridization may be used as the capturing molecule.

In Step S610, immobilization of the capturing molecules on the metasurface 110 may be promoted by using a reagent such as EDC (1-ethyl-3-(3-dimethylaminopropyl)-carbodiimide hydrochloride)/NHS(N-hydroxysuccinimide) thereby activating the binding end of the capturing molecule. After passage of the capturing molecule-containing solution, a buffer or the like is passed for removal of suspended molecules and washing the inside of the channel. Note here that the capturing molecule-containing solution through the microchannel may be collected in a waste collection vessel (not shown).

In Step S620, the solution containing the biomolecule to be inspected is passed through the microchannel 130 in the inspection chip 100 with the result that when the target biomolecule is present in the biomolecules to be inspected, it is specifically captured by the capturing molecules immobilized on the metasurface 110 in Step S610, and immobilized on the metasurface 110. When the target biomolecule is the aforesaid protein such as immunoglobulin and the capturing molecule is the aforesaid antibody binding protein or avidin derivative, the target biomolecule is captured while it passes through the microchannel 130. Again, the solution containing the biomolecule to be inspected through the microchannel may be corrected in a waste collection vessel.

When the target biomolecule *per se* is excited by excitation light to be described later to emit fluorescence or when the target biomolecule is labeled with a fluorescent label that is excited with excitation light to be described later to emit fluorescence, the process goes to Step S630. Prior to going to Step S630, it is again preferable to pass a buffer or the like through the microchannel for removal of suspended molecules or nonspecific adsorbed molecules.

When the target biomolecule does neither emit fluorescence by itself nor is labeled with a fluorescent label, on the other hand, a solution containing antibody molecules (also called the secondary antibodies) that are fluorescently labeled may be passed through the microchannel 130 in the inspection chip 100 subsequent to Step S620. Such secondary antibodies are capable of binding specifically to the target biomolecule by cross-reactivity and may be used in place of the fluorescent label of the target biomolecule itself. Secondary antibodies are also capable of binding each other; they are expected to contribute more to fluorescence amplification.

For the fluorescent labels for labeling the target biomolecule or the secondary antibody, those known in the art may be used; they may be selected from the group consisting of DyLight, fluorescein, acrylodan, rhodamine, BODIPY, acridine orange, eosin, pyrene, PyMPO, Alexa Fluor 488, Alexa Fluor 532, Alexa Fluor 546, Alexa Fluor 568, Alexa Fluor 594, Alexa Fluor 555, Alexa Fluor 633, Alexa Fluor 647, Alexa Fluor 660, and Alexa Fluor 680, each having excitation wavelengths and fluorescence wavelengths well known in the art.

The target biomolecule or the secondary antibody may be labeled by the known method set forth in commercially available fluorescent labeling kits.

In Step S630, excitation light is incident from the second substrate 140 side of the inspection chip 100 and illuminates on the metasurface 110. Such excitation light is emitted from the light source 510 of the detection apparatus 500 shown in Fig. 5. If the target biomolecule is present on the metasurface 110, fluorescence is then emitted by irradiation of excitation light on the basis of the target biomolecule itself, the fluorescent label provided on the target biomolecule or the fluorescent labeled on the secondary antibody bound to the target biomolecule. According to the present invention, it is thus unnecessary to locate a prism as described in Patent Publication 2 thereby exciting the target biomolecule with evanescent light.

Upon emission of fluorescence, it resonates between the first substrate 120 and second substrate 140 of the inspection chip 100, and then radiates toward the second substrate 140 side, as shown in Fig. 2.

In Step S640, light radiating from the second substrate 140 side is detected. In this step, the radiating fluorescence is detected using the detection means 520 of the detection apparatus 500 depicted in Fig. 5.

In Step S650, whether or not the target biomolecules are present in the biomolecules to be inspected is determined on the basis of the detected fluorescence. When the detected light is fluorescence based on the aforesaid fluorescent label or the target biomolecule itself, it can be judged as being present. When the detected light is not fluorescence on the basis of the aforesaid fluorescent label or the target biomolecule itself, it can be judged as being absent. Such judgment may be carried out either by a computer 540 incorporated in the detection apparatus 500 or spectra or optical images displayed on the output apparatus 550.

The inspection chip 100 used in the steps as described above can be repeatedly used if the capturing molecules, etc. immobilized on the metasurface 110 are removed. Removal of proteins such as the capturing molecules is preferably carried out by ultrasonic washing using an alkali aqueous solution.

By way of exemplification but not by way of limitation, the present invention will now be explained in more details with reference to specific examples.

**Examples**

Metasurface

Silicon nanorod structures, and gold complementary stacked structures were prepared as the metasurface.

To prepare a metasurface comprising the silicon nanorod structure, there was a Si substrate 410 (Fig. 4) provided, comprising a silicon on an insulator (SOI: Silicon On Insulator) and a buried oxide film (BOX: Buried Oxide) layer. This substrate is called the SOI substrate overall. The SOI layer had a thickness of 200 nm, and the BOX layer had a thickness of 375 nm. Note here that the SOI layer comprised crystalline Si, and the BOX layer comprised $SiO_2$.

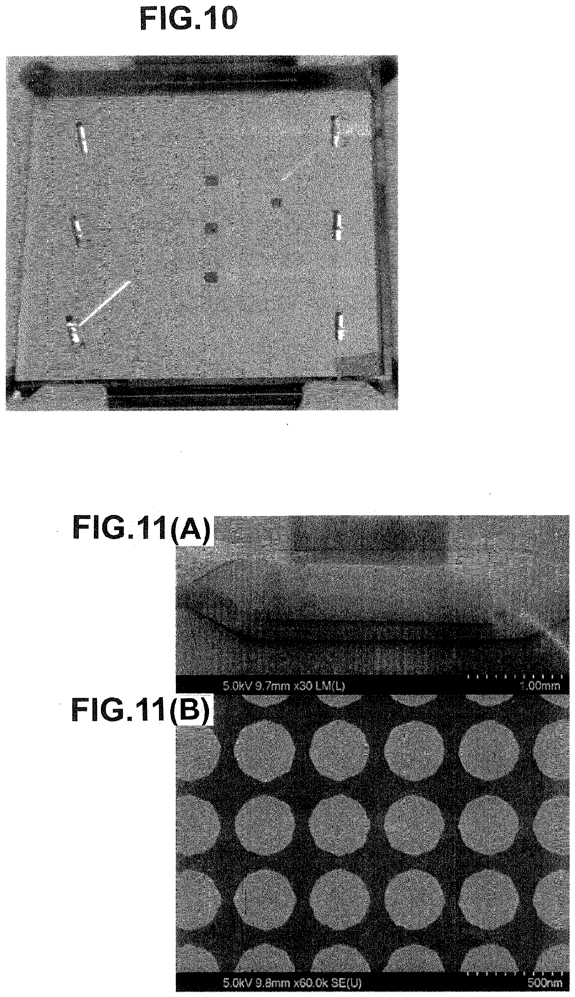

A cylindrical periodic array having a period $\Lambda_2$ of 400 nm and a diameter $D_2$ of 300 nm, and a cylindrical periodic array having a period $\Lambda_2$ of 300 nm and a diameter $D_2$ of 220 nm were designed, respectively. Electron beam lithography (EBL) was applied to the SOI substrate to prepare a resist mask. Anisotropic and selective etching was carried out by BOSCH processing for etching of the SOI layer alone. Consequently, there were a variety of silicon nanorod structures obtained with a period $\Lambda_2$ of 400 nm and a diameter $D_2$ of 302 nm, 316 nm or 320 nm, and a silicon nanorod structure having a period $\Lambda_2$ of 300 nm and a diameter $D_2$ of 236 nm was obtained as well. Thus, plural silicon nanorods having a height equal to the thickness of the SOI layer were prepared. After BOSCH processing, the remaining mask was eliminated by $O_2$ plasma. The metasurface defined by the obtained silicon nanorod structure was observed by a scanning electron microscope (SEM by Hitachi, Model No. SU8230), with the results shown in Fig. 11(B).

A metasurface having a gold complementary stacked structure comprised a Si slab material 320 on a Si substrate 310 (Fig. 3) including a buried oxide film 340 (Fig. 3), and gold (Au) as a metallic material 330. Holes formed in the slab material 320 had a diameter $D_1$ of any one of 302 nm, 291 nm, 250 nm or 315 nm and a depth of 230 nm, and a period $\Lambda_1$ of 410.5 nm in one direction and a period of 710 nm in a direction orthogonal thereto. These periods stand for lengths of the short and long sides of rectangular unit cells formed by the periodically arrayed holes.

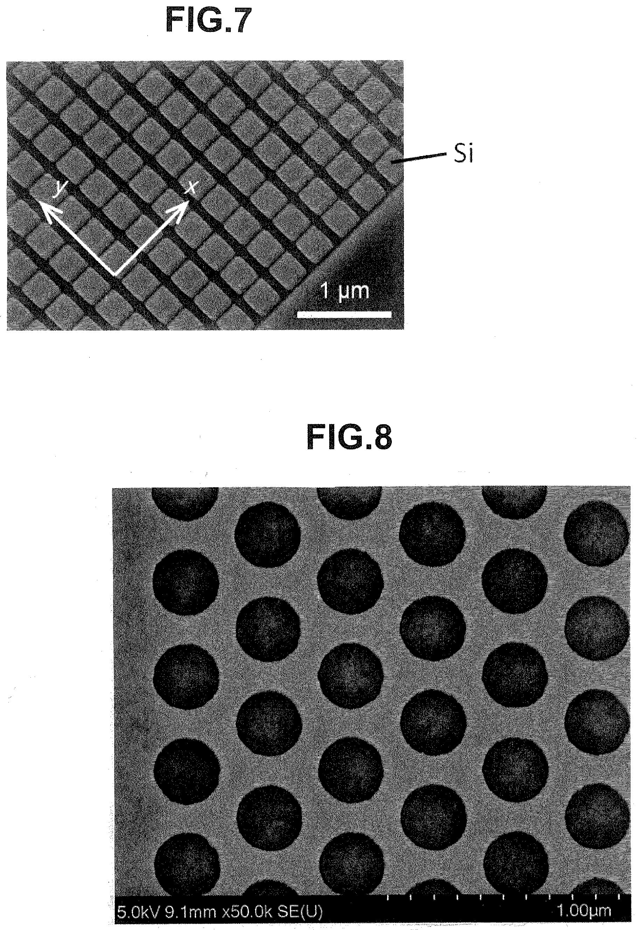

The metasurface defined by the gold complementary stacked structure was prepared by procedures or steps similar to those of Example 1 set forth in Patent Publication 4. The metasurface defined by the obtained gold complementary stacked structure was observed by SEM with the results shown in Fig. 8.

Fig. 7 is an SEM image indicative of the metasurface of the silicon nanorod structure, and Fig. 8 is an SEM metasurface indicative of the gold complementary stacked structure.

Fig. 7 shows a nanorod structure wherein silicon nanorods in a quadrangular column form having a height of 200 nm and a section perpendicular to the height direction, defined by a square having one side length of 320 nm, are arrayed in a period of 400 nm. Fig. 8 shows a top view image of the gold complementary stacked structure comprising round holes periodically arrayed in a period of 410.5/710 nm with a diameter of 291 nm. This structure was found to have a height of 230 nm.

To identify that the silicon nanorod structure of Fig. 7 induces fluorescence enhancement, rhodamine 590 (R590) molecules were dispersed on the SOI substrate having said structure and a comparative flat Si substrate, respectively, which were subsequently irradiated with incident laser light having a wavelength of 532 nm to measure and compare the then emitted fluorescent spectra. As a result, the SOI substrate having a silicon nanorod structure has been found to exhibit emission intensity enhancement in an emission peak wavelength of 590 nm. This result is also in agreement with the report of Non-Patent Publication 1 that the silicon nanorod structure provides a metasurface capable of developing fluorescence enhancement in the respective wavelength ranges of 550 nm to 570 nm inclusive, 580 nm to 600 nm inclusive, and 650 nm to 690 nm inclusive.

Referring to the gold complementary stacked structure, Non-Patent Publication 3 also teaches that emission intensity is found to be enhanced in the wavelength ranges of 550 nm to 600 nm inclusive, 650 nm to 700 nm inclusive and 750 nm to 800 nm inclusive by measurement and comparison of fluorescent spectra observed when R590 molecules were dispersed in a gold complementary stacked structure (with a period of 410.5/710 nm and a hole diameter of 265 nm) and a comparative flat Si substrate, respectively. Thus, the gold complementary stacked structure (with a period of 410.5/710 nm and a circle diameter of 265 nm) has also been found to provide a metasurface capable of developing fluorescence enhancement in a range of 550 nm to 600 nm inclusive.

To identify that the gold complementary stacked structure develops fluorescence enhancement with respect to other wavelengths, too, IR783 molecules were dispersed in a gold complementary stacked structure (with a period of 410.5/710 nm and a hole diameter of 250 nm) and a comparative flat Si substrate, respectively, for measurement and comparison of fluorescent spectra. As a result, the substrate having a gold complementary stacked structure has been found to develop emission intensity enhancement in a wavelength of 850 nm to 900 nm inclusive. From this, it has been identified that the gold complementary stacked structure (with a period of 410.5/710 nm and a circle diameter of 250 nm) also provides a metasurface capable of fluorescence enhancement in a range of 850 nm to 900 nm inclusive.

Samples

Protein AG-cys or cys-streptavidin (Click Biosystems, PRO1001 or PRO1005) was used for the capturing molecule, and anti-avidin antibody (Abcam, ab53494) was used for the capturing antibody.

Used for the target biomolecules were immunoglobulin G (IgG, Abcam, ab206202), p53 antibody (Novus Biologicals, NB200-171), mouse monoclonal p53 antibody (Abcam, ab16465), CEA (Abcam, ab742), and p53 antibody (Abcam, ab210717) labeled with fluorescent molecule (AF 555, Alexa Fluor 555), goat anti-mouse IgG antibody (Abcam, ab150114) labeled with fluorescent molecule (AF 555, Alexa Fluor 555), goat anti-rabbit IgG antibody (Abcam, ab150078) labeled with fluorescent molecule (AF 555, Alexa Fluor 555) or IgG (Novus Biologicals, NBP1-76055) labeled with fluorescent molecule (DL555, DyLight 555).

Biotin Labeling Kit (Wako Pure Chemistry Industries 347-90891), Fluorescent Molecule HiLyte 555 (HL555) Labeling Kit (Wako Pure Chemistry Industries 348-90141) and Fluorescent Molecule Indocyanine Green (ICG) Labeling Kit (Wako Pure Chemistry Industries 345-91431) were also used to provide the target biomolecule with fluorescent and biotin labels, if required.

In the following Examples 1 to 8 and Comparative Examples 1 and 2, the aforesaid metasurfaces were used to prepare inspection chips, and the aforesaid samples were used for fluorescence measurements.

Example 1

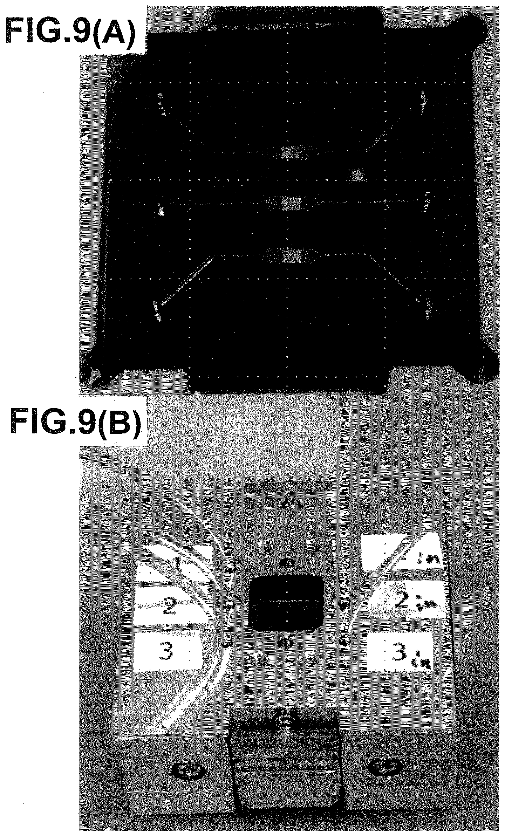

In Example 1, the second substrate 140 (Fig. 1) made of polydimethylsiloxane (PDMS) and having a microchannel (having a height D of 30 μm) was stuck, by way of self-sucking, to the first substrate 120 (Fig. 1) of the metasurface 110 (Fig. 1) having a cylindrical silicon nanorod structure having a period of 400 nm and a diameter of 320 nm, and formed an inspection chip. An appearance of the inspection chip is shown in Fig. 9(A), and an appearance of flow channel apparatus incorporating the chip is shown in Fig. 9(B). Note here that PDMS is known to have an average transmission of visible light of 60% or higher.

Fig. 9 shows the inspection chip of Example 1 and the appearance of the flow channel apparatus incorporating the chip.

As described above, the inspection chip is set up by self-sucking of the second substrate 140 (Fig. 1) made of polydimethylsiloxane (PDMS) with a microchannel therethrough to the first substrate 120 (Fig. 1) including the metasurface 110 (Fig. 1) having a silicon nanorod structure, contributing much more to simplification of the inspection chip preparation process.

As can be seen from Fig. 9(A), the inspection chip has three microchannels, each incorporating the metasurface 110. Such configuration allows for immobilization of the target biomolecule and detection of fluorescence to take place in parallel to a plurality of target biomolecules depending on the number of microchannels. The rectangular metasurface is positioned in the center of the horizontal hexagon in Fig. 9(A). The apparatus is configured such that a sample, etc. are fed from an inlet positioned right in Fig. 9(A) and waste is discharged out of an outlet positioned left in Fig. 9(A). Fig. 9(B) is a photograph of the flow channel apparatus comprising connection tubes through which a reagent flows in the inspection chip from outside and the reagent is discharged out, and a cover adapted to cover the inspection chip. This cover also serves as an inspection chip retainer to prevent any liquid leakage in the inspection chip where the channels are sealed up only by self-sucking of the first substrate with the second substrate.

The inspection chip and flow channel apparatus according to Example 1 were used to inspect the fluorescently labeled target biomolecule as described just below. PBS was passed from the inlets of the connection tubes through the microchannels in the inspection chip for 4 minutes, rinsing the channels. Then, a PBS diluted solution of protein AG-cys (having a concentration of 146 μg/mL) as the capturing molecules was continuously passed for 20 minutes, whereupon the microchannels were again rinsed inside with PBS for 4 minutes. The flow rate was 10 μL/min. Then, 400 μL of PBS diluted IgG molecules fluorescently labeled with HL 555 (having a concentration of 5 ng/mL, 0.5 ng/mL, and 0.05 ng/mL) were intermittently (ON/OFF=1/3) passed through the respective microchannels in the inspection chip. The average flow rate was 8 μL/min. Thereafter, the microchannels were rinsed with PBS for removal of suspended molecules and non-specific adsorbed molecules, and the microchannels were then dried inside by a flow of dry nitrogen.

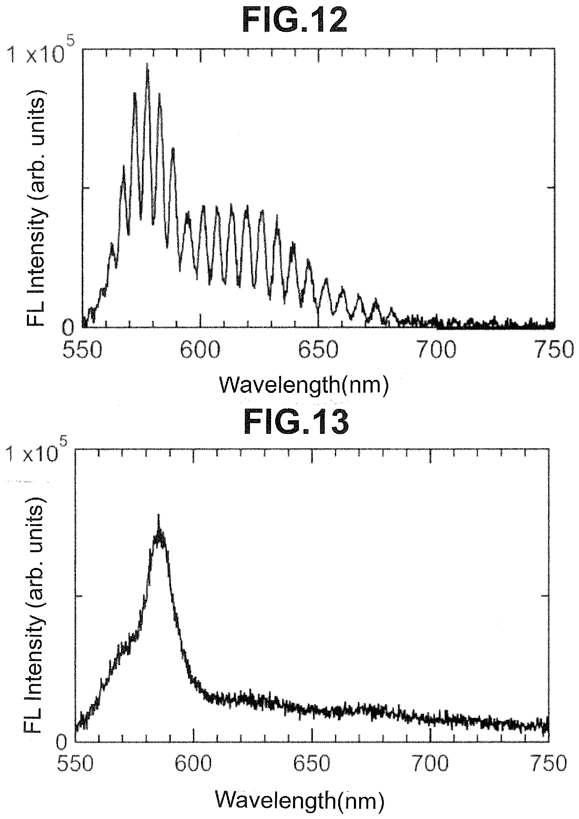

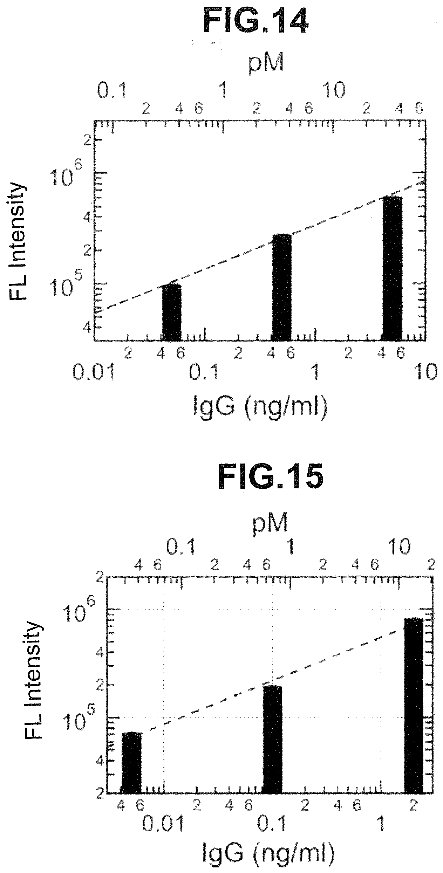

The inspection chip having the microchannels dried inside was loaded in the detection apparatus shown in Fig. 5 for fluorescence measurement. A laser adapted to emit light of 532 nm in wavelength (Model No. Action 532Q-0050 available from ATOK Co., Ltd.) was used as the light source 510, and an imaging spectrometer (Model No. IsoPlane 160-ProEM1024HS available from Princeton-Instruments Co., Ltd.) was used as the detection means 520. The power of laser light irradiated on the metasurface was 0.17 mW, and the measuring time was 20 seconds. The results of observation of the post-measurement inspection chip by SEM are shown in Fig. 11. Fluorescent spectra measured on the metasurface with a passage of the diluted solution (of 5 ng/mL in concentration) of the target biomolecule fluorescently labeled with HL555 are shown in Fig. 12, and relationships between the concentration of the target biomolecule obtained from fluorescent spectra and the intensity of fluorescence are depicted in Fig. 14.

Example 2

In Example 2, Example 1 was repeated with the exception of using the metasurface having a cylindrical silicon nanorod structure of 300 nm in period and 236 nm in diameter, thereby configuring an inspection chip and flow channel apparatus. The obtained inspection chip had an appearance similar to that shown in Fig. 9(A), and the flow channel apparatus had an appearance similar to that shown in Fig. 9(B).

In Example 2, an IgG molecule fluorescently labeled with AF555 was used as the target biomolecule. Two hundred fifty (250) μL of a PBS-diluted solution of this target biomolecule (having a concentration of 2 ng/ml, 0.1 ng/ml, and 0.005 ng/ml) were passed through the respective microchannels in the inspection chip. The flow rate was 8.3 μl/min. Rinsing was carried out with PBS as in Example 1. Relationships between the target biomolecule concentration and the fluorescence intensity are shown in Fig. 15.

Example 3

In Example 3, by way of self-sucking, the second substrate 140 (Fig. 1) formed of PDMS and including microchannels (of 30 μm in height) was stuck to the first substrate 120 (Fig. 1) having the metasurface 110 (Fig. 1) having a gold complementary stacked structure having a period of 410.5/710 nm and a hole diameter of 302 nm to configure an inspection chip, whose outer appearance is shown in Fig. 10.

Fig. 10 is illustrative of the outer appearance of the inspection chip of Example 3.

As described above, the inspection chip is configured by self-sucking of the second substrate 140 (Fig. 1) formed of PDMS and having microchannels to the first substrate 120 (Fig. 1) having the metasurface 110 (Fig. 1) comprising a gold complementary stacked structure. Thus, the steps of preparing inspection chips were significantly simplified.

As can be seen from Fig. 10, the inspection chip has three microchannels, each having the metasurface located inside. Such configuration allows for immobilization of the target biomolecule and inspection of fluorescence to take place in parallel with respect to a plurality of target biomolecules depending on the number of microchannels. The rectangular metasurface is positioned in the center of the horizontal hexagon in Fig. 9(B). The apparatus is configured such that a sample, etc. are fed from an inlet positioned right in Fig. 9(B) and waste is discharged out of an outlet positioned left in Fig. 9(B). The inspection chip was held down with the cover shown in Fig. 9(B), and connected to connection tubes into a flow channel apparatus.

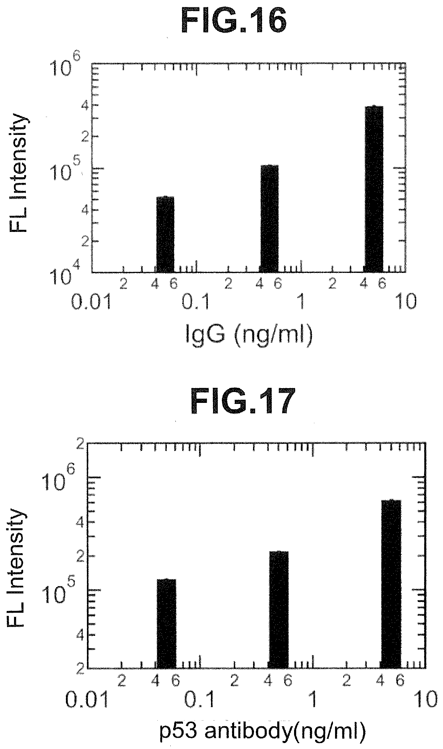

Example 1 was repeated with the exception that IgG molecules fluorescently labeled with HL555 was further labeled with biotin for detection of the target bio-molecules. Relationships between the target biomolecule concentration and the fluorescence intensity are shown in Fig. 16.

Example 4

In Example 4, an inspection chip was configured as in Example 1 with the exception of using the first substrate having a cylindrical silicon nanorod structure of 316 nm in diameter. The inspection chip had an appearance similar to that shown in Fig. 9(A). The target bio-molecules were detected as in Example 1 with the exception that p53 antibodies, which are tumor markers, fluorescently labeled with AF555 were used as the target biomolecules. Relationships between the target biomolecule concentration and the fluorescence intensity are shown in Fig. 17.

Example 5

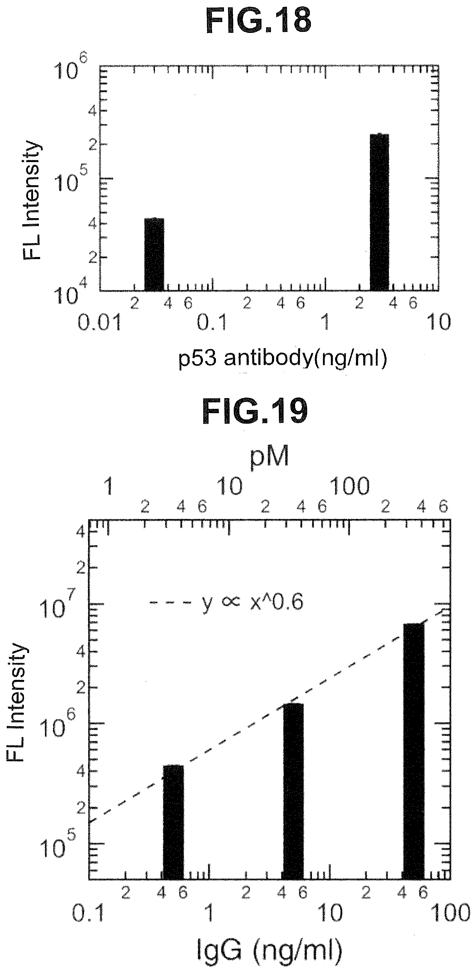

In Example 5, an inspection chip was configured as in Example 3 with the exception of using the first substrate including the metasurface having a gold complementary stacked structure having a hole diameter of 291 nm. The inspection chip had an outer appearance similar to that shown in Fig. 10. The target biomolecules were detected as in Example 1 with the exception that cys-streptavidin was used as the capturing molecule, and a p53 antibody fluorescently labeled with HL555 and further labeled with biotin was used as the target biomolecule. Relationships between the target biomolecule concentrations and the fluorescence intensities are shown in Fig. 18.

Example 6

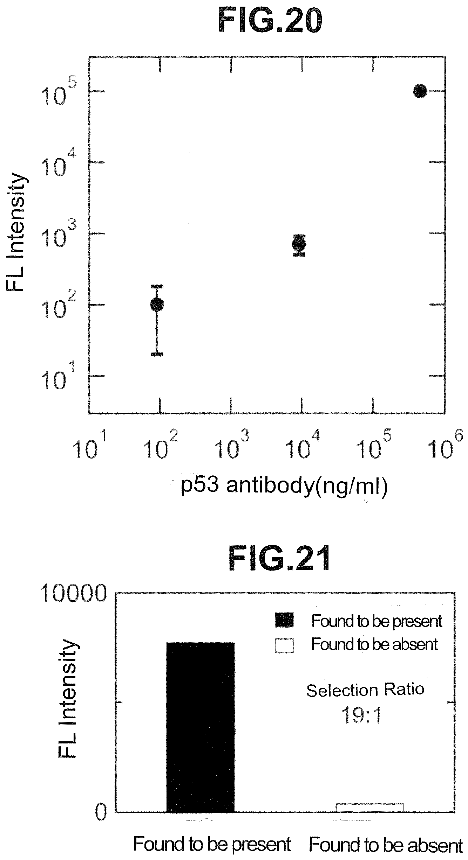

In Example 6, the efficiencies of detection of the target biomolecules depending on the presence or absence of capturing antibodies were compared with the use of the inspection chip and flow channel apparatus prepared in Example 5.
First of all, the target biomolecules were detected as in Example 1 with the exception that the biomolecule concentration was 50 ng/mL. Prior to the passage of the target biomolecules through the PBS-rinsed flow channel apparatus of Example 5, they were detected as in Example 1 with the exception that a solution diluted with anti-biotin antibody (of 10 μg/mL in concentration) was passed as the capturing antibody for 25 minutes. Fluorescence intensities were compared depending on the presence or absence of the capturing antibodies, with the results shown in Fig. 21.

Example 7

In Example 7, the inspection chip and flow channel apparatus were configured as in Example 1 with the exception of using the first substrate having the metasurface including a cylindrical silicon nanorod structure of 400 nm in period and 302 nm in diameter was used, and the microchannels formed through the second substrate made of PDMS had each a microchannel height of 15 μm. The inspection chip had an outer appearance similar to that shown in Fig. 9(A), and the flow channel apparatus had an outer appearance similar to that shown in Fig. 9(B). The obtained flow channel apparatus was used to detect the target biomolecules as in Example 1. IgG molecules fluorescently labeled with AF555 and further labeled with biotin were used as the target biomolecules. The IgG concentrations used herein were 5 ng/ml, 0.33 ng/ml and 0.022 ng/mL, respectively.

Comparative Example 1

In Comparative Example 1, the inspection chip and flow channel apparatus were constructed for detection of the target biomolecules as in Example 1 with the exception of using the metasurface having a cylindrical silicon nanorod structure of 400 nm in period and 302 nm in diameter. The concentrations of the target biomolecules used herein were 50 ng/mL, 5 ng/mL and 0.5 ng/mL. At the time of measuring the detection of fluorescence, the second substrate formed of PDMS was removed to measure only the single substrate (the first substrate), and the ensuing measurements were compared with those of the examples of performing fluorescence detection through a micro-resonator. Fluorescence spectra obtained from the target biomolecules in a concentration of 50 ng/mL are shown in Fig. 13. Relationships between the biomolecule concentrations and the fluorescence intensities are shown in Fig. 19.

Comparative Example 2

In Comparative Example 2, only the first substrate having the metasurface including a gold complementary stacked structure having a hole diameter of 250 nm was used. Five hundreds (500) ng/mL of a PBS-diluted solution of protein A were added dropwise to the metasurface as the capturing molecules, and the metasurface was then held for 1 hour in a room temperature/high-humidity environment for immobilization of the molecules to the surface, followed by rinsing with PBS. Then, two (2) μL of a PBS-diluted solution of IgG molecules labeled with ICG (of 90 ng/mL, 9000 ng/mL and 450000 ng/mL in concentration) were added dropwise to the metasurface of each of separately provided substrates, followed by a 2-hour incubation under a room temperature/high-humidity environment. Thereafter, it was rinsed with PBS for removal of suspended molecules, etc. and the metasurface was dried by a flow of dry nitrogen.
The target biomolecules were detected as in Example 1. Relationships between the target biomolecule concentrations and the fluorescence intensities are shown in Fig. 20.

Example 8

In Example 8, the inspection chip and flow channel apparatus were assembled as in Example 3 with the exception of using the first substrate having a metasurface comprising a gold complementary stacked structure having a hole diameter of 315 nm. The inspection chip had an outer appearance similar to that shown in Fig. 10.

The inspection chip and flow channel apparatus of Example 8 were used to detect the target biomolecules that were not fluorescently labeled, as follows. PBS was passed from the inlet of the connection tube in the flow channel apparatus through the microchannels for 5 minutes to fill the liquid in the channels. Then, a PBS-diluted solution of protein AG-cys (of 146 µg/mL in concentration) was passed as the capturing molecules for 23 minutes. The flow rate used herein was 11 µl/min. Thereafter, the microchannels were rinsed inside with PBS for 6 minutes. Subsequently, 360 µL of a PBS-diluted solution (of 1 ng/mL in concentration) of p53 antibodies without fluorescent labeling were allowed to flow through the microchannels. The flow rate used herein was 7.7 µl/mL. Again, the microchannels were rinsed inside with PBS for 10 minutes. Then, 400 µl (of 9.1 µg/mL in concentration) of goat polyclonal and anti-rabbit IgG fluorescently labeled with DL555 were passed as the secondary antibodies. Then, the microchannels were then rinsed with PBS for removal of suspended molecules and non-specific absorbed molecules, and dry nitrogen was permitted to flow through the microchannels for drying inside.

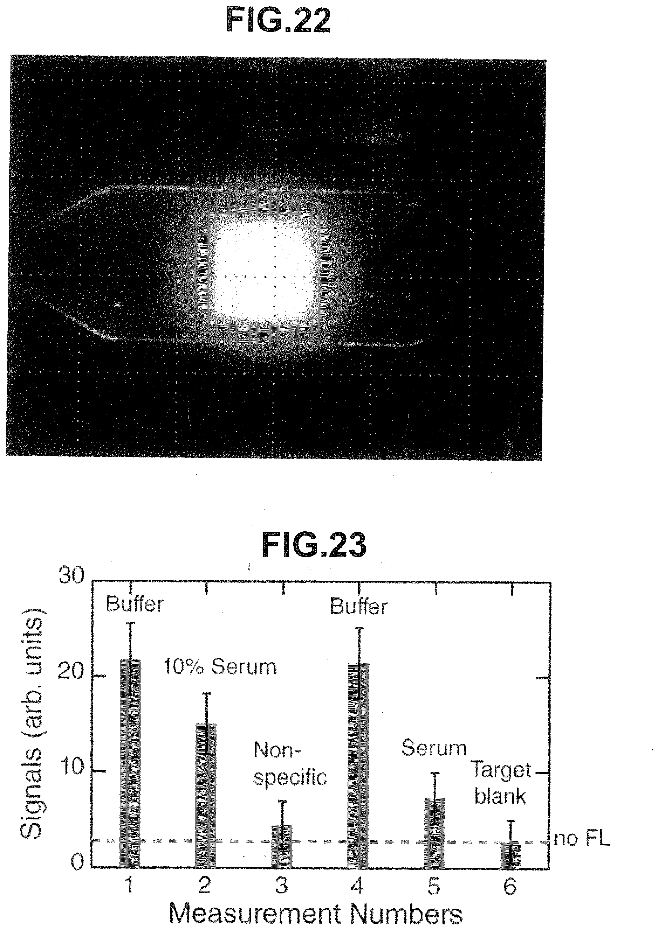

The inspection chip with the microchannels dried inside was loaded in the detection apparatus shown in Fig. 5 for measurement of fluorescent images. A light emitting diode of wavelength 532 nm band was used as the light source 510, and a CCD camera (made by Lumenera Co., Ltd., Model No. INFINITY-3S) was used as the detection means. The measuring time used herein was 2 seconds. A fluorescent image taken by the CCD camera is shown in Fig. 22.

To make it easy to understand the aforesaid results, the inspection chips of Examples 1 to 8 and Comparative Examples 1 and 2 are summed up together with the experimental conditions in Tables 1 and 2.

Table 1: List of Inspection Chips

| Examples | 1st Substrate | | |
| --- | --- | --- | --- |
| | Metasurface | Material | Diameter (nm) |
| Ex. 1 | Nanorod | Si | 320 |
| Ex. 2 | Nanorod | Si | 236 |
| Ex. 3 | Complementary stacked | Au/SOI | 302 |
| Ex. 4 | Nanorod | Si | 316 |
| Ex. 5 | Complementary stacked | Au/SOI | 291 |
| Ex. 6 | Complementary stacked | Au/SOI | 291 |
| Ex. 7 | Nanorod | Si | 302 |
| Comp. Ex 1 | Nanorod | Si | 302 |
| Comp. Ex 2 | Complementary stacked | Au-SOI | 250 |
| Ex. 8 | Complementary stacked | Au/SOI | 315 |

| Examples | 1st Substrate | | |
| --- | --- | --- | --- |
| | Period (nm) | Height (nm) | Range of Fluorescence Enhancement (nm) |
| Ex. 1 | 400 | 200 | 550-570, 580-600, 650-690 |

| | | | | |
|---|---|---|---|---|
| Ex. 2 | 300 | 200 | 550-570, 580-600, 650-690 | Table 1 (continued) |
| Ex. 3 | 410.5/710 | 230 | 550-600, 650-700, 750-800 | |
| Ex. 4 | 400 | 200 | 550-570, 580-600, 650-690 | |
| Ex. 5 | 410.5/710 | 230 | 550-600, 650-700, 750-800 | |
| Ex. 6 | 410.5/710 | 230 | 550-600, 650-700, 750-800 | |
| Ex. 7 | 400 | 200 | 550-570, 580-600, 650-690 | |
| Comp. Ex 1 | 400 | 200 | 550-570, 580-600, 650-690 | |
| Comp. Ex 2 | 410.5/700 | 230 | 850-900 | |
| Ex. 8 | 410.5/700 | 230 | 550-600, 650-700, 750-800 | |

| Examples | 2nd Substrate Material | Distance D (μm) |
|---|---|---|
| Ex. 1 | PDMS | 30 |
| Ex. 2 | PDMS | 30 |
| Ex. 3 | PDMS | 30 |
| Ex. 4 | PDMS | 30 |
| Ex. 5 | PDMS | 30 |
| Ex. 6 | PDMS | 30 |
| Ex. 7 | PDMS | 15 |
| Comp. Ex. 1 | – | – |
| Comp. Ex. 2 | – | – |
| Ex. 8 | PDMS | 30 |

Table 2: List of the Experimental Conditions

| Example | Capturing Molecule | Capturing Antibody | Biomolecule | Fluorescent Label |
|---|---|---|---|---|
| Example 1 | Protein AG· cys | - | IgG | HL555 |
| Example 2 | Protein AG· cys | - | IgG | AF555 |
| Example 3 | Protein AG· cys | Anti-biotin antibody | IgG[*1] | HL555 |
| Example 4 | Protein AG· cys | - | p53 antibody | AF555 |
| Example 5 | Cys· streptavidin | - | p53 antibody | HL555 |

(continued)

| | | | | |
|---|---|---|---|---|
| Example 6 | Protein AG· cys | Anti-biotin antibody | IgG[*1] | HL555 |
| Example 7 | Protein AG· cys | Anti-biotin antibody | IgG[*1] | AF555 |
| Comp. Ex. 1 | Protein AG· cys | - | IgG | HL555 |
| Comp. Ex. 2 | Protein AG· cys | - | IgG | ICG |
| Example 8 | Protein AG· cys | - | IgG[*2] | DL555[*3] |

*1: Fluorescently labeled biomolecule labeled with biotin
*2: Biomolecule having no fluorescent label
*3: Fluorescently labeled secondary antibody

Fig. 11 is SEM images of condition of the post-inspection chip of Example 1.

Fig. 11(A) is a low-magnification SEM image showing the outer appearance of the inspection chip according to Example 1 after inspection, including the metasurface. The boundaries of the microchannels are so clearly imaged that differences in the substrate surface state due to the presence or absence of fluid flows are distinctively observed. This result supports that there is no fluid leakage at all. According to Fig. 11(B) that is an SEM image of the upper metasurface, there is no aggregation of capturing molecules, biomolecules, etc. observed at all, suggesting that the capturing molecules, biomolecules, etc. can be immobilized by the steps of Example 1 on the outermost surface individually in a molecular level without forming any aggregation composite on the metasurface of the inspection chip. Although not shown, no fluid leakage was also observed in the inspection chips of other examples.

Fig. 12 is indicative of emission spectra observed with the use of the inspection chip according to Example 1.

Fig. 13 is indicative of emission spectra observed with the use of the inspection chip according to Comparative Example 1.

Fig. 12 is indicative of emission spectra measured after the passage of the target biomolecules having a HL555-labeled IgG molecule concentration of 5 ng/ml through the inspection chip of Example 1. According to Fig. 12, there is an emission peak observed in the vicinity of 570 nm wavelength, and there is a secondary peak of emission observed in the vicinity of 610 nm, indicating that this emission is fluorescence based on HL555 fluorescently labeled on IgG. From this, it has been identified that the wavelength region where the inventive inspection chip develops fluorescence enhancement exists certainly in the fluorescent wavelength range of HL555, and that the fluorescently labeled target biomolecules can be detected by the fluorescence detection process comprising the steps shown in Fig. 6.

Further, as shown in Fig. 12, the emission spectra according to Example 1 shows an interference fringe having a period as short as about 10 nm. As shown in Fig. 13, however, the emission spectra according to Comparative Example 1 did not show similar interference fringes. Although not shown, the emission spectra according to Comparative Example 2 did not show any interference fringe derived from the microresonator either.

From the results of Fig. 12 and the results of Comparative Examples 1 and 2, it has been shown that the use of the second substrate 140 (Fig. 1) having a microchannel as the inspection chip causes fluorescence to resonate between the metasurface 110 (Fig. 1) of the first substrate 120 (Fig. 1) and the second substrate 140 (Fig. 1), followed by radiation through the second substrate, and that the microresonator is defined by the first substrate having a metasurface, the second substrate having a microchannel and a space of the μm order between these substrates.

Although not shown, emission spectra observed through other inspection chips according to Examples 2 to 8 also show an interference fringe derived from the microresonator, indicating that the first substrate having a metasurface and the second substrate having a microchannel function as a microresonator from which fluorescence is radiated through them. From these results, it has been understood that the substrate-to-substrate distance suitable for resonance of fluorescence between the first and second substrates is 15 μm to 50 μm inclusive.

Fig. 14 shows a standard curve obtained from fluorescence measurement according to Example 1.

Fig. 15 shows a standard curve obtained from fluorescence measurement according to Example 2.

Fig. 16 shows a standard curve obtained from fluorescence measurement according to Example 3.

Fig. 17 shows a standard curve obtained from fluorescence measurement according to Example 4.

Fig. 18 shows a standard curve obtained from fluorescence measurement according to Example 5.

Fig. 19 shows a standard curve obtained from fluorescence measurement according to Comparative Example 1.

Fig. 20 shows a standard curve obtained from fluorescence measurement according to Comparative Example 2.

The standard curve of Fig. 14 is obtained by integrating signal intensities having wavelengths of 590 nm to 598 nm inclusive from the fluorescence spectra in Example 1 and plotting them with respect to the concentration of the target biomolecules. The standard curves of Figs. 15 to 20 are again obtained by integrating signal intensities from the fluorescence spectra according to Examples 2-5 and Comparative Examples 1-2 and plotting them with respect to the concentration of the target biomolecules.

According to Fig. 14, when the inspection chip of Example 1 was used, fluorescence signals were observed even in a concentration as low as 0.05 ng/ml. As indicated by a black dotted line, the fluorescence intensity responded to the IgG molecular concentration in a power law, and a certain plateau suggestive of detection limits (a constant value) was not yet reached in the measurement concentration.

As can be seen from Fig. 15, when the inspection chip of Example 2 was used, a signal was detected even in a concentration of the target biomolecules as low as 0.005 ng/mL (or 0.034 pM where M is molar, i.e., mol/liter). This indicates that the inventive inspection chip forming the microresonator is capable of efficient detection of the target biomolecules. It has further been understood that the attenuation rate of a signal y with respect to a concentration x can be approximated by $y = x^{0.4}$ indicated by the black dotted line in Fig. 15. This indicates that the signal attenuation is extremely low with respect to concentration, suggesting that even target biomolecules in a much lower concentration (of 1 pg/mL order as an example) can be fluorescently detected by the inventive inspection chip. In other words, given the inventive inspection chip, the target biomolecules can be detected with a detection sensitivity about 34000 times as high as a certified value of 17 ng/mL (HRP-labeled IgG, Abcam, ab6721) which is said to be detectable by ELISA method.

As can be seen from Figs. 16 to 18 (Examples 3 to 5), fluorescence signals were observed in a low-concentration region as in Fig. 14 (Example 1) so that the target biomolecules could be detected. Although not shown, when the inspection chip of Example 7 was used, it was also possible to detect the target biomolecules in a low-concentration region. From this, it has been shown that the inventive inspection chip, because of locating the first substrate having a metasurface having a metal complementary stacked or nanorod structure in opposition to the second substrate having a microchannel to form a microresonator, is suitable for fluorescence detection of low-concentration biomolecules regardless of species.

As can be seen from Fig. 19 (Comparative Example 1), on the other hand, the attenuation rate of signal y with respect to concentration x was represented by $y \propto x^{0.6}$, and that the signal attenuated abruptly with a decrease in the target biomolecule concentration. From this, it has been appreciated that fluorescence radiation control by the inventive inspection chip is effective for detection of low-concentration target biomolecules.

As can be seen from Fig. 20 (Comparative Example 2), the signal attenuated more abruptly with a decrease in the target biomolecule concentration. From comparisons of Figs. 14 to 18 with Fig. 20, it has been understood that the capturing molecules are efficiently immobilized on the metasurface by way of sample delivery through the microchannel in the inventive inspection chip so that the target biomolecules can be captured in a more efficient manner.

Fig. 21 is indicative of comparisons of fluorescence intensities due to the presence or absence of the capturing antibodies in Example 6.

According to Fig. 21, the use of an anti-biotin antibody as the capturing antibody made detection intensity 19 times as strong. From this, it has been appreciated that when the inventive inspection chip is used for detection of the target biomolecules by a fluorescence detection method, it is possible to use a capturing antibody inducing biotin/anti-biotin binding reaction thereby promoting specific binding, rendering more sensitive detection possible.

Fig. 22 is a fluorescence image of the inspection chip in Example 8.

Although Fig. 22 is shown in a gray scale, a region shown in white corresponds to yellow light. From this, it has been identified that if the inventive inspection chip is used according to the procedure or steps shown in Fig. 6, even the target biomolecule, if not fluorescently labeled, can be detected by use of a fluorescently labeled secondary antibody.

Example 9

In Example 9, the same inspection chip and flow channel apparatus as in Example 3 were used to study reproducibility and the influence of a buffer.

According to the same steps as in Example 1, fluorescence detection was carried out by using cys-streptavidin as the capturing molecule and a diluted solution of IgG molecule fluorescently labeled with HL555 (10 ng/mL in concentration, NS buffer, 10% serum, whole serum) as the target biomolecule. It is here noted that for comparison purposes, fluorescence detection was carried out without recourse to the capturing molecule, and both the capturing molecule and the target biomolecule.

Summary of the Experimental Examples

Experiment 1)

The capturing molecules were passed through the flow channel apparatus as in Example 1, whereupon the target biomolecules diluted with an NS buffer (ab193972 made by Abcam Co., Ltd.) were passed at an average flow rate of 7 μL/min. for 30 minutes.

Experiment 2)

Experiment 1 was repeated except that the target biomolecules were diluted with a 90% NS buffer and 10% human serum.

Experiment 3)

Experiment 1 was repeated except that the capturing molecules were not passed through the flow channel apparatus beforehand.

Experiment 4)

On the day after measurement carried out in Experiment 1, the metasurface prepared on the same design was used to carry out experimentation similar to Experiment 1 for the purpose of identifying reproducibility.

Experiment 5)

After passage of the capturing molecules through the flow channel apparatus as in Example 1, the target biomolecules diluted with 100% human serum were passed at an average flow rate of 4 $\mu$L/min. for 50 minutes.

Experiment 6)

To determine a background signal level, only the NS buffer was passed through the flow channel apparatus at an average flow rate of 7 $\mu$L/min. for 30 minutes without passing both the capturing molecules and the target biomolecules.
While the microchannel was filled up with a PBS rinsing liquid, fluorescence measurement was carried out using LED light as excitation light with a wavelength range limitation by a band pass filter having a 527 nm band, and a fluorescent image was taken by a CCD camera through a fluorescent band pass filter having a 591 nm band.
The results of fluorescence measurement obtained in this way are shown in Fig. 23.
Measurement Nos. 1 to 6 in Fig. 23 are corresponding to the aforesaid Experiments 1 to 6, respectively. From a comparison of Experiment 1 with Experiment 4, it has been understood that the inventive inspection chip excels in reproducibility.
As can be seen from the results of Experiment 2, even when the inventive inspection chip was used with 10% human serum as the buffer, good enough fluorescence could be detected, although there was a slight decrease in intensity found as compared with the NS buffer such as PBS. More surprisingly, even when whole serum was used as the buffer, there was a fluorescence intensity high enough to be differentiated from Experiment 3 (with no capturing molecule) or Experiment 6 (with a background signal level), although there was a drop of fluorescence intensity. From this, it has been shown that the inventive inspection chip can be used with serum as is the case with the NS buffer.

Example 10

In Example 10, the same inspection chip and flow channel apparatus as in Example 3 were used for indirect detection of mouse monoclonal p53 antibodies.
First of all, a solution of cys-streptavidin diluted with PBS (having a concentration of 20 $\mu$g/mL) was passed as the capturing molecule at a flow rate of 11 to 12 $\mu$L/min. for 30 minutes. Then, the microchannels were rinsed inside with PBS for 10 minutes. Then, biotin-labeled mouse monoclonal p53 antibodies (channel 1), NS buffer (channel 2) and biotin-labeled mouse monoclonal p53 antibodies (channel 3) were passed through each of three channels of the flow channel apparatus at a flow rate of 11 to 13 $\mu$l/min for 30 minutes, with channel 2 as blank. The biotin-labeled mouse monoclonal p53 antibodies were diluted with the NS buffer down to 5 $\mu$g/mL.
Subsequently, the three channels were washed with PBS for 15 minutes, followed by passage of a solution of fluorescent molecule-labeled goat anti-mouse AF555-IgG diluted with the NS buffer through channels 1 and 2, and a solution of fluorescent molecule-labeled goat anti-rabbit AF555-IgG diluted with the NS buffer through channel 3 for 30 minutes, respectively. The fluorescent molecule-labeled goat anti-mouse antibody and goat anti-rabbit antibody were each a secondary antibody. These diluted solutions had a concentration of 100 ng/mL, and were passed at an average flow rate of 11 to 12 $\mu$L/min. Thereafter, the microchannels were rinsed with PBS for 10 minutes for removal of suspended molecules and non-specific adsorbed molecules, and dry nitrogen was allowed to flow through the microchannels for drying, followed by fluorescence measurement as in Example 9.

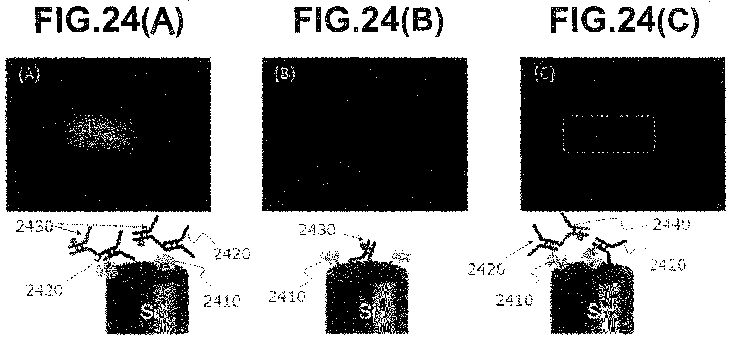

Fig. 24 shows fluorescent images of each channel and schematic views of each state where biomolecules remain immobilized in place.

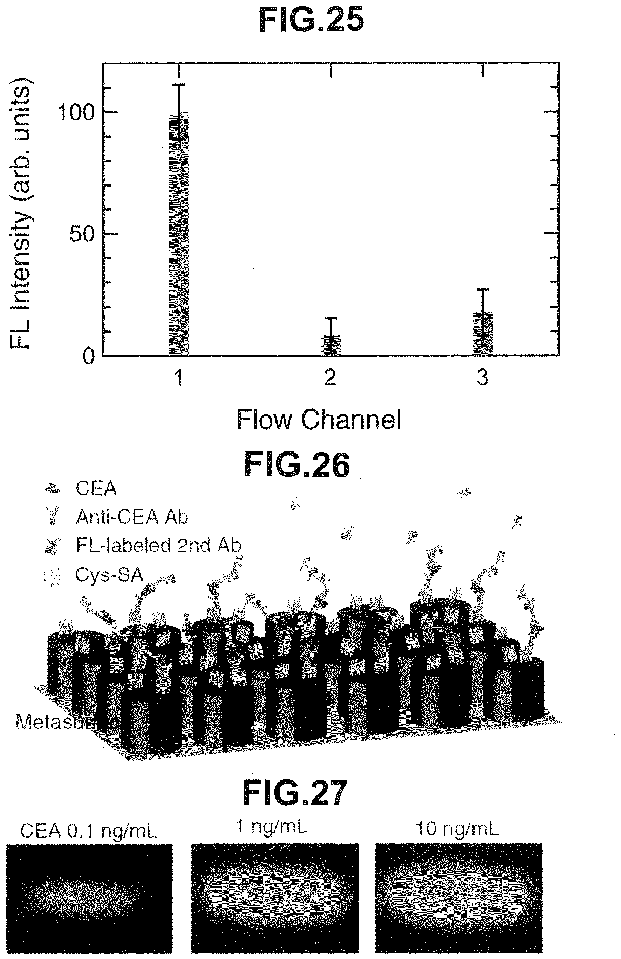

Fig. 25 shows comparisons of fluorescence intensities in Example 10.

Figs. 24(A) to (C) show the fluorescent images and schematic binding states of each inspection chip located in channels 1 to 3 running through the flow channel apparatus. As can be seen from Fig. 24(A), capturing molecule (cys-streptavidin) 2410 is immobilized to the metasurface of the inspection chip, mouse monoclonal p53 antibody 2420 is immobilized to the capturing molecule 2410, and secondary antibody (fluorescence molecule-labeled goat anti-mouse AF555-IgG) 2430 is immobilized to the mouse monoclonal p53 antibody 2420 in channel 1. A region indicated here in white emits flourescence based on the secondary antibody.

As can be seen from Fig. 24(C), capturing molecule (cys-streptavidin) 2410 is immobilized to the metasurface of the inspection chip, mouse monoclonal p53 antibody 2420 is immobilized to the capturing molecule 2410 and secondary antibody (fluorescence molecule-labeled goat anti-rabbit AF555-IgG) 2440 is immobilized to the mouse monoclonal p53 antibody 2420 in channel 3 as is the case with cannel 1. A region indicated here by a white dotted line emits fluorescence based on the secondary antibody. This emission takes place due to mouse/rabbit cross-reaction.

As no target biomolecule (mouse monoclonal p53 antibody) is passed through channel 2, on the other hand, capturing molecule (cys-streptavidin) 2410 immobilized as is the case with other channels, and accidentally and non-specifically immobilized secondary antibody (fluorescence molecule-labeled goat anti-mouse AF555-IgG) 2430 are merely staying on the metasurface of the inspection chip as can be seen from Fig. 24(B). As there was no or little light emission observed in channel 2, the inventive inspection chip would ensure that false signals stemming from the nonspecific adsorption of the secondary antibodies may be made small to the full.

From a comparison of channel 1 with channel 2 in Fig. 25, it has been identified that when fluorescently unlabeled target biomolecules are present on the metasurface, they can be specifically detected by use of fluorescently labeled secondary antibodies.

From a comparison of channel 1 with channel 3 in Fig. 25, it has been understood that cross reaction taking place between the mouse monoclonal p53 antibody and the goat anti-rabbit antibody is so limited that fluorescence is held down. This suggests that by measurement of fluorescence intensity, the p53 antibody can selectively and specifically be detected for each animal species.

Example 11

In Example 11, the same inspection chip and flow channel apparatus as in Example 3 were used for indirect detection of CEA in a sandwich assay configuration. The CEA is a tumor marker used for medical diagnosis.

First of all, a solution of cys-streptavidin diluted with PBS (having a concentration of 20 $\mu$g/mL) was passed as the capturing molecule through the flow channel apparatus for 50 minutes to immobilize the capturing molecule to the metasurface of the inspection chip. Then, rabbit monoclonal, i.e., an anti-CEA antibody (ab229074 available from Abcam) was provided, and a natural protein CEA (ab742 available from Abcam) was provided as the target biomolecule. The anti-CEA antibody used here was labeled with biotin, and not.

The concentrations of the biotin-labeled anti-CEA antibody and the anti-CEA antibody were each adjusted to 100 ng/mL using the NS buffer. The CEA was adjusted to three concentrations 30 ng/mL, 3 ng/mL and 0.3 ng/mL, using the NS buffer.

A reaction solution of the biotin-labeled anti-CEA antibody (100 $\mu$L), the target biomolecule CEA (100 $\mu$L) in the respective concentrations and the anti-CEA antibody (100 $\mu$L) was incubated for 60 minutes at room temperature (299K) and 400 rpm to prepare three sandwiched composite cocktails of anti-CEA antibody/target biomolecule CEA/biotin-labeled anti-CEA antibody. The final concentration of the target CEA in each cocktail was 10 ng/mL, 1 ng/mL, and 0.1 ng/mL.

Each cocktail was passed through the respective channels of the flow channel apparatus at an average flow rate of 10 $\mu$L/min for 28 minutes, and a washing buffer in an ELISA kit (ab195215 available from Abcam) was passed through them for 10 minutes, followed by a 10-minute rinsing with PBS.

Subsequently, fluorescence molecule-labeled AF555-IgG was passed as the secondary antibody through the respective flow channels at an average flow rate of 9 $\mu$L/min for 30 minutes. AF555-IgG used here was a goat-polyclonal anti-rabbit antibody (ab150078 available from Abcam). After the respective channels were rinsed with PBS for 10 minutes, fluorescence measurement was carried out as in Example 9.

Fig. 26 is a schematic view indicative of the state of molecules on the metasurface of the inspection chip in Example 11.

As can be seen from Fig. 26, the capturing molecule (cys-streptavidin) is immobilized onto the metasurface, and the sandwiched composite of anti-CEA antibody/target bio-molecule CEA/biotin-labeled anti-CEA antibody is immobilized to the capturing molecule. Although the target biomolecule CEA is label-free (or has no fluorescent label), it is possible to detect it with fluorescence.

Further, as can be seen from Fig. 26, AF555-IgG is immobilized as the secondary antibody to the biotin-labeled anti-CEA antibody of the sandwiched composite. The secondary antibodies, because of being polyclonal, are bonded together for fluorescence enhancement.

Fig. 27 is a set of fluorescent images of CEA in various concentrations in Example 11.

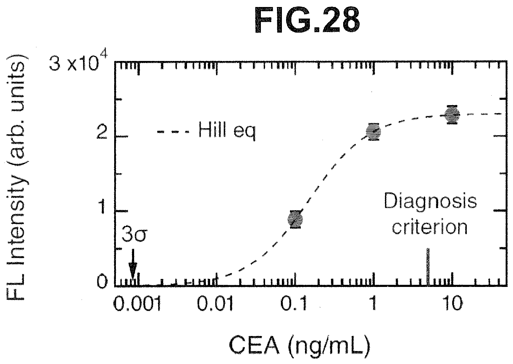

Fig. 28 is a diagram showing the dependency of fluorescence intensities on CEA concentration,obtained from fluorescence measurement in Example 11.

In Fig. 27 given on a gray scale, a region indicated in white emits yellow light. As can be seen from Fig. 27, the intensity of fluorescence of the metasurface (in a rectangular region shown here) increases with an increase in the target biomolecule CEA concentration in the sandwiched composite. It should be noted that the second antibodies were also flown in regions of the inspection chip other than the metasurface; however, any significant fluorescence is not found in regions other than the metasurface. From this, it has been anticipated that the secondary antibodies would be specifically bound to the anti-CEA antibodies of the sandwiched composite.

In Fig. 28, the dotted line stands for a fitting curve using the following Hill equation.

$$y = y_0 + (S - y_0) x^n / (x^n + K_D^n)$$

where y is a fluorescence intensity, $y_0$ is a background level upon fluorescence measurement, S is a saturated signal intensity, x is a concentration of target biomolecules, n is a degree of cooperative reaction, and $K_D$ is a dissociation constant.

As can be seen from Fig. 28, it has been appreciated that the profile conforms well to the fitting curve, and that because there is a region where fluorescence intensity decreases linearly from the saturated region, high-sensitivity detection is achieved. Although the present criterion for CEA medical diagnosis is 5 ng/mL, it has been shown that if the inventive inspection chip is used, CEA can then be detected in even lower concentrations so that quantitative results can be provided with high precision.

Further, the CEA concentration $3\sigma$($\sigma$:standard deviation)-away from the CEA concentration turning to a zero signal was found to estimate the limit of detection (LOD) statistically with the result that LOD was 0.85 pg/mL (indicated by $3\sigma$ in Fig. 28), suggesting that when the background is ideally held down, the target biomolecule CEA having a very low concentration can be detected by fluorescence measurement.

**Industrial Applicability**

The biomolecular inspection chip for fluorescence detection according to the present invention can be applied to every technique configured to detect biomolecules by fluorescence detection methods. If the inventive inspection chip is used, it is then possible to detect biomolecules in a concentration as low as, e.g., 1 pg/mL order, with high precision.

**Explanation of the Reference Numerals**

100: Biomolecular inspection chip for fluorescence detection
110, 300, 400: Metasurface
120: First substrate
130: Microchannel
140: Second substrate
310, 410: Substrate material
320: Slab material
330: Metallic material
340: Surface layer
350: Hole
360: Hole's side wall
420: Nanorod
500: Detection apparatus
510: Light source
520: Detection means
530: Filter
540: Computer
550: Output apparatus

**Claims**

1.  A biomolecular inspection chip (100) for fluorescence detection, comprising:

    a first substrate (120) having a metasurface (110), and
    a second substrate (140) positioned in opposition to the first substrate, wherein
    a microchannel (130) is formed by the first substrate and the second substrate,
    the metasurface is an artificial nanostructure surface, includes gaps for efficient immobilization of a biomolecule to be detected and induces fluorescence enhancement in a wavelength range of fluorescence emitted by the biomolecule,
    the second substrate is formed of a material transparent to visible light or near infrared light, and the fluorescence resonates between the first substrate and second substrate,
    the metasurface comprises a metal complementary stacked structure;
    the metal complementary stacked structure includes a substrate material, a slab material positioned on a surface of the substrate material, and a metallic material positioned on at least the slab material,
    the substrate material comprises a surface layer in contact with at least the slab material,
    the slab material comprises a material having a refractive index higher than that of the surface layer and includes a plurality of periodically arranged holes extending from its surface to the surface layer of the substrate material, and
    the metallic material is positioned on a surface of the slab material and the surface layer of the substrate material defining bottom surfaces of the plurality of holes, respectively; and
    the material of the second substrate is at least one selected from silicone-based resin, (meth)acrylic-based resin, epoxy-based resin, styrene-based resin, polycarbonate, ester-based resin, acrylonitrile-butadienestyrene resin, polyamide, and cycloolefin polymer, and
    the microchannel formed by the first substrate and the second substrate has a height of 15 $\mu$m to 50 $\mu$m inclusive.

2.  The inspection chip according to claim 1, wherein the metallic material is a substance having a complex dielectric constant approximate to that of a Drude metal.

3.  The inspection chip according to claim 2, wherein the substance having a complex dielectric constant approximate to that of a Drude metal is selected from the group consisting of gold (Au), silver (Ag), copper (Cu), aluminum (Al), platinum (Pt), titanium (Ti), nickel (Ni), and alloys of these metals.

4.  The inspection chip according to any one of claims 1 to 3, wherein the plurality of holes comprise round holes having two or more different diameters or prismatic holes having two or more one-side lengths, and/or are arranged in two or more different periods.

5.  The inspection chip according to any one of claims 1 to 4, wherein the plurality of holes have a period of 300 nm to 1000 nm inclusive,

    the plurality of holes have a diameter or one-side length of 100 nm to 500 nm inclusive, and
    the slab material has a thickness of 100 nm to 2 $\mu$m inclusive.

6.  A biomolecular inspection chip (100) for fluorescence detection, comprising:

    a first substrate (120) having a metasurface (110), and
    a second substrate (140) positioned in opposition to the first substrate, wherein
    a microchannel (130) is formed by the first substrate and the second substrate,
    the metasurface is an artificial nanostructure surface, includes gaps for efficient immobilization of a biomolecule to be detected and induces fluorescence enhancement in a wavelength range of fluorescence emitted by the biomolecule,
    the second substrate is formed of a material transparent to visible light or near infrared light, and the fluorescence resonates between the first substrate and second substrate,
    the metasurface comprises a nanorod structure,
    the nanorod structure comprises a substrate material and a plurality of periodical, upright nanorods on the surface of the substrate material, and
    the substrate material is formed of a material having a refractive index lower than that of the plurality of nanorods; and
    the material of the second substrate is at least one selected from silicone-based resin, (meth)acrylic-based resin, epoxy-based resin, styrene-based resin, polycarbonate, ester-based resin, acrylonitrile-butadienestyrene resin, polyamide, and cycloolefin polymer, and
the microchannel formed by the first substrate and the second substrate has a height of 15 μm to 50 μm inclusive.

7. The inspection chip according to claim 6, wherein the substrate material comprises a material having a refractive index of 1 to 1.6 inclusive, and the plurality of nanorods comprise a semiconductor or a dielectric material having a refractive index of 2 to 5 inclusive.

8. The inspection chip according to claim 7, wherein the semiconductor or dielectric material is selected from the group consisting of silicon, germanium, gallium nitride, and titanium dioxide.

9. The inspection chip according to any one of claims 6 to 8,

   wherein the plurality of nanorods have a period of 300 nm to 1000 nm inclusive,
   the plurality of nanorods have a diameter or one-side length of 100 nm to 500 nm inclusive, and
   the plurality of nanorods have a height of 100 nm to 2 μm inclusive.

10. The inspection chip according to any one of claims 6 to 9, wherein the plurality of nanorods comprise round columns having two or more different diameters or prismatic columns having two or more different one-side lengths, and/or are arranged in two or more different periods.

11. The inspection chip according to any of claims 1 to 10, wherein the fluorescence enhancement comprises intensity enhancement of light having a wavelength in a visible light region or a near infrared light region.

12. The inspection chip according to any of claims 1 to 11, wherein the material of the second substrate is polydimethylsiloxane (PDMS).


**Patentansprüche**

1. Biomolekularer Inspektionschip (100) für eine Fluoreszenzerfassung, der aufweist:

   ein erstes Substrat (120), das eine Metaoberfläche (110) aufweist, und
   ein zweites Substrat (140), das gegenüber dem ersten Substrat positioniert ist, wobei
   ein Mikrokanal (130) durch das erste Substrat und das zweite Substrat gebildet ist,
   die Metaoberfläche eine künstliche Nanostrukturoberfläche ist, Lücken für eine effiziente Immobilisierung eines zu erfassenden Biomoleküls enthält, und eine Fluoreszenzverstärkung in einem Wellenlängenbereich einer Fluoreszenz, die durch das Biomolekül emittiert wird, induziert,
   das zweite Substrat aus einem Material, das für sichtbares Licht oder nah-infrarotes Licht transparent ist, gebildet ist, und die Fluoreszenz zwischen dem ersten Substrat und dem zweiten Substrat resoniert,
   die Metaoberfläche eine komplementär gestapelte Metallstruktur aufweist;
   die komplementär gestapelte Metallstruktur ein Substratmaterial, ein Plattenmaterial, das auf einer Oberfläche des Substratmaterials positioniert ist, und ein metallisches Material, das zumindest auf dem Plattenmaterial positioniert ist, enthält,
   das Substratmaterial eine Oberflächenschicht, die in Kontakt mit zumindest dem Plattenmaterial ist, aufweist,
   das Plattenmaterial ein Material aufweist, das einen Brechungsindex, der größer als der der Oberflächenschicht ist, aufweist und eine Vielzahl von periodisch angeordneten Löchern, die sich von seiner Oberfläche zu der Oberflächenschicht des Substratmaterials erstrecken, enthält, und
   das metallische Material auf einer Oberfläche des Plattenmaterials bzw. der Oberflächenschicht des Substratmaterials, das untere Oberflächen der Vielzahl von Löchern definiert, positioniert ist; und
   das Material des zweiten Substrats aus zumindest einem aus einem silikonbasierten Harz, einem (meth) akrylbasierten Harz, einem epoxidbasierten Harz, einem styrenbasierten Harz, einem Polycarbonat, einem esterbasierten Harz, einem Akrylonitril-Butadien-Styren-Harz, einem Polyamid und einem Cycloolefinpolymer ausgewählt ist, und
   der Mikrokanal, der durch das erste Substrat und das zweite Substrat gebildet ist, eine Höhe von 15 μm bis 50 μm inklusive aufweist.

2. Inspektionschip nach Anspruch 1, wobei das metallische Material eine Substanz ist, die eine komplexe dielektrische Konstante aufweist, die näherungsweise die eines Drudemetalls ist.

3.  Inspektionschip nach Anspruch 2, wobei die Substanz, die eine komplexe dielektrische Konstante, die näherungsweise die eines Drudemetalls ist, aus der Gruppe ausgewählt ist, die aus Gold (Au), Silber (Ag), Kupfer (Cu), Aluminium (Al), Platin (Pt), Titan (Ti), Nickel (Ni) und Legierungen dieser Metalle besteht, ausgewählt ist.

4.  Inspektionschip nach einem der Ansprüche 1 bis 3, wobei die Vielzahl der Löcher runde Löcher, die zwei oder mehr verschiedene Durchmesser haben, oder prismatische Löcher, die zwei oder mehr Einseitenlängen haben, aufweisen, und/oder in zwei oder mehr verschiedenen Perioden angebracht sind.

5.  Inspektionschip nach einem der Ansprüche 1 bis 4, wobei die Vielzahl der Löcher eine Periode von 300 nm bis 1000 nm einschließlich aufweisen,

    die Vielzahl der Löcher einen Durchmesser oder eine Einseitenlänge von 100 nm bis 500 nm einschließlich aufweisen, und
    das Plattenmaterial eine Dicke von 100 nm bis 2 $\mu$m einschließlich aufweist.

6.  Biomolekularer Inspektionschip (100) für eine Fluoreszenzerfassung, der aufweist:

    ein erstes Substrat (120), das eine Metaoberfläche (110) aufweist, und
    ein zweites Substrat (140), das gegenüber dem ersten Substrat positioniert ist, wobei
    ein Mikrokanal (130) durch das erste Substrat und das zweite Substrat gebildet ist,
    die Metaoberfläche eine künstliche Nanostrukturoberfläche ist, Lücken für eine effiziente Immobilisierung eines zu erfassenden Biomoleküls enthält, und eine Fluoreszenzverstärkung in einem Wellenlängenbereich einer Fluoreszenz, die durch das Biomolekül emittiert wird, induziert,
    das zweite Substrat aus einem Material, das transparent für sichtbares Licht oder nah-infrarotes Licht ist, gebildet ist, und die Fluoreszenz zwischen dem ersten Substrat und dem zweiten Substrat resoniert,
    die Metaoberfläche eine Nanostäbchenstruktur aufweist,
    die Nanostäbchenstruktur ein Substratmaterial und eine Vielzahl von periodischen aufrechten Nanostäbchen auf der Oberfläche des Substratmaterials aufweist, und
    das Substratmaterial aus einem Material, das einen Brechungsindex aufweist, der niedriger als der der Vielzahl von Nanostäbchen ist, gebildet ist; und
    das Material des zweiten Substrats zumindest eines, das aus einem silikonbasiertem Harz, einem (meth) akrylbasiertem Harz, einem epoxidbasiertem Harz, einem styrenbasiertem Harz, einem Polycarbonat, einem esterbasiertem Harz, einem Akrylonitril-Butadien-Styren-Harz, einem Polyamid, und einem Cycloolefinpolymer ausgewählt ist, ist, und
    der Mikrokanal, der durch das erste Substrat und das zweite Substrat gebildet wird, eine Höhe von 15 $\mu$m bis 50 $\mu$m einschließlich aufweist.

7.  Inspektionschip nach Anspruch 6, wobei das Substratmaterial ein Material aufweist, das einen Brechungsindex von 1 bis 1,6 einschließlich aufweist, und die Vielzahl von Nanostäbchen einen Halbleiter oder ein dielektrisches Material, das einen Brechungsindex von 2 bis 5 einschließlich aufweist, aufweist.

8.  Inspektionschip nach Anspruch 7, wobei der Halbleiter oder das dielektrische Material aus der Gruppe, die aus Silizium, Germanium, Galliumnitrid und Titandioxid besteht, ausgewählt ist.

9.  Inspektionschip nach einem der Ansprüche 6 bis 8, wobei die Vielzahl der Nanostäbchen eine Periode von 300 nm bis 1000 nm einschließlich aufweisen,

    die Vielzahl der Nanostäbchen einen Durchmesser oder eine Einseitenlänge von 100 nm bis 500 nm einschließlich aufweisen, und
    die Vielzahl der Nanostäbchen eine Höhe von 100 nm bis 2 $\mu$m einschließlich aufweisen.

10. Inspektionschip nach einem der Anschlüsse 6 bis 9, wobei die Vielzahl der Nanostäbchen runde Säulen, die zwei oder mehr verschiedene Durchmesser haben, oder prismatische Säulen, die zwei oder mehr verschiedene Einseitenlängen haben, aufweisen, und/oder in zwei oder mehr verschiedenen Perioden angebracht sind.

11. Inspektionschip nach einem der Ansprüche 1 bis 10, wobei die Fluoreszenzverstärkung eine Intensitätsverstärkung von Licht, das eine Wellenlänge in einem sichtbaren Lichtbereich oder einem Nah-Infrarotlichtbereich aufweist, aufweist.

12. Inspektionschip nach einem der Ansprüche 1 bis 11, wobei das Material des zweiten Substrats Polydimethylsiloxan (PDMS) ist.

**Revendications**

1. Puce d'inspection biomoléculaire (100) pour la détection de fluorescence, comprenant :

   un premier substrat (120) comportant une métasurface (110), et
   un deuxième substrat (140) positionné en face du premier substrat, dans laquelle
   un microcanal (130) est formé par le premier substrat et le deuxième substrat,
   la métasurface est une surface de nanostructures artificielles, comporte des espaces pour l'immobilisation efficace d'une biomolécule à détecter et induit une augmentation de la fluorescence dans une plage de longueurs d'onde de fluorescence émise par la biomolécule,
   le deuxième substrat est constitué d'un matériau transparent à la lumière visible ou au rayonnement proche infrarouge, et la fluorescence résonne entre le premier substrat et le deuxième substrat,
   la métasurface comprend une structure empilée métallique complémentaire ;
   la structure empilée métallique complémentaire comporte un matériau substrat, un matériau en plaque positionné sur une surface du matériau substrat, et un matériau métallique positionné au moins sur le matériau en plaque,
   le matériau substrat comprend une couche de surface en contact au moins avec le matériau en plaque,
   le matériau en plaque comprend un matériau ayant un indice de réfraction supérieur à celui de la couche de surface et comporte une pluralité de trous disposés avec une périodicité s'étendant depuis sa surface jusqu'à la couche de surface du matériau substrat, et
   le matériau métallique est positionné sur une surface du matériau en plaque et la couche de surface du matériau substrat, définissant des surfaces inférieures de la pluralité de trous, respectivement ; et
   le matériau du deuxième substrat en est au moins un choisi parmi une résine à base de silicone, une résine (méth) acrylique, une résine époxy, une résine à base de styrène, le polycarbonate, une résine à base d'ester, une résine acrylonitrile-butadiène-styrène, le polyamide, et un polymère cyclooléfinique, et
   le microcanal formé par le premier substrat et le deuxième substrat présente une hauteur de 15 $\mu$m à 50 $\mu$m inclus.

2. Puce d'inspection selon la revendication 1, dans laquelle le matériau métallique est une substance ayant une constante diélectrique complexe se rapprochant de celle d'un métal de Drude.

3. Puce d'inspection selon la revendication 2, dans laquelle la substance ayant une constante diélectrique complexe se rapprochant de celle d'un métal de Drude est choisie dans le groupe constitué par l'or (Au), l'argent (Ag), le cuivre (Cu), l'aluminium (Al), le platine (Pt), le titane (Ti), le nickel (Ni), et les alliages de ces métaux.

4. Puce d'inspection selon l'une quelconque des revendications 1 à 3, dans laquelle la pluralité de trous comprennent des trous ronds présentant au moins deux diamètres différents ou des trous prismatiques présentant au moins deux longueurs d'un côté, et/ou sont disposés selon au moins deux périodes.

5. Puce d'inspection selon l'une quelconque des revendications 1 à 4, dans laquelle la pluralité de trous présentent une période de 300 nm à 1000 nm inclus,

   la pluralité de trous présentent un diamètre ou une longueur d'un côté de 100 nm à 500 nm inclus, et
   le matériau en plaque présente une épaisseur de 100 nm à 2 $\mu$m inclus.

6. Puce d'inspection biomoléculaire (100) pour la détection de fluorescence, comprenant :

   un premier substrat (120) comportant une métasurface (110), et
   un deuxième substrat (140) positionné en face du premier substrat, dans laquelle
   un microcanal (130) est formé par le premier substrat et le deuxième substrat,
   la métasurface est une surface de nanostructures artificielles, comporte des espaces pour l'immobilisation efficace d'une biomolécule à détecter et induit une augmentation de la fluorescence dans une plage de longueurs d'onde de fluorescence émise par la biomolécule,
le deuxième substrat est constitué d'un matériau transparent à la lumière visible ou au rayonnement proche infrarouge, et la fluorescence résonne entre le premier substrat et le deuxième substrat,
la métasurface comprend une structure de nanotiges,
la structure de nanotiges comprend un matériau substrat et une pluralité de nanotiges verticales périodiques sur la surface du matériau substrat, et
le matériau substrat est constitué d'un matériau ayant un indice de réfraction inférieur à celui de la pluralité de nanotiges ; et
le matériau du deuxième substrat en est au moins un choisi parmi une résine à base de silicone, une résine (méth) acrylique, une résine époxy, une résine à base de styrène, le polycarbonate, une résine à base d'ester, une résine acrylonitrile-butadiène-styrène, le polyamide, et un polymère cyclooléfinique, et
le microcanal formé par le premier substrat et le deuxième substrat présente une hauteur de 15 $\mu$m à 50 $\mu$m inclus.

7. Puce d'inspection selon la revendication 6, dans laquelle le matériau substrat comprend un matériau ayant un indice de réfraction de 1 à 1,6 inclus, et la pluralité de nanotiges comprennent un matériau semi-conducteur ou diélectrique ayant un indice de réfraction de 2 à 5 inclus.

8. Puce d'inspection selon la revendication 7, dans laquelle le matériau semi-conducteur ou diélectrique est choisi dans le groupe constitué par le silicium, le germanium, le nitrure de gallium, et le dioxyde de titane.

9. Puce d'inspection selon l'une quelconque des revendications 6 à 8,

dans laquelle la pluralité de nanotiges présentent une période de 300 nm à 1000 nm inclus,
la pluralité de nanotiges présentent un diamètre ou une longueur d'un côté de 100 nm à 500 nm inclus, et
la pluralité de nanotiges présentent une hauteur de 100 nm à 2 $\mu$m inclus.

10. Puce d'inspection selon l'une quelconque des revendications 6 à 9, dans laquelle la pluralité de nanotiges comprennent des colonnes rondes présentant au moins deux diamètres différents ou des colonnes prismatiques présentant au moins deux longueurs d'un côté différentes, et/ou sont disposées selon au moins deux périodes.

11. Puce d'inspection selon l'une quelconque des revendications 1 à 10, dans laquelle l'augmentation de la fluorescence comprend l'augmentation de l'intensité de la lumière ayant une longueur d'onde dans une région de lumière visible ou une région de rayonnement proche infrarouge.

12. Puce d'inspection selon l'une quelconque des revendications 1 à 11, dans laquelle le matériau du deuxième substrat est le polydiméthylsiloxane (PDMS).

# FIG.1

# FIG.2

Fluorescently labeled biomolecule

Capturing antibody

Captured molecule

~5 nm

D

140

120

110

# FIG.3

# FIG.4

# FIG.5

# FIG.6

Start

S610 — The capturing molecules are passed through the microchannels for anchorage to the metasurface.

S620 — The biomolecules to be detected are passed through the microchannels to anchor them to the metasurface together with the capturing molecules

S630 — Irradiation of excitation light

S640 — The presence or absence of the target biomolecules are judged on the basis of the detected light.

S650 — Fluorescence from the inspection chip is detected

Over

# FIG.7

# FIG.8

5.0kV 9.1mm x50.0k SE(U)                                    1.00μm

FIG.9(A)

FIG.9(B)

## FIG.10

## FIG.11(A)

## FIG.11(B)

## FIG.12

## FIG.13

# FIG.14

# FIG.15

# FIG.16

# FIG.17

## FIG.18

p53 antibody(ng/ml)

## FIG.19

pM

- - - - y ∝ x^0.6

IgG (ng/ml)

# FIG.20

# FIG.21

# FIG.22

# FIG.23

FIG.24(A)   FIG.24(B)   FIG.24(C)

# FIG.25

# FIG.26

- CEA
- Anti-CEA Ab
- FL-labeled 2nd Ab
- Cys-SA

Metasurface

# FIG.27

CEA 0.1 ng/mL     1 ng/mL     10 ng/mL

# FIG.28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 3657790 A
- JP 2011220996 A
- JP 2006177878 A
- JP 2017173084 A
- WO 2011002010 A1
- US 2014206101 A1

**Non-patent literature cited in the description**

- **MASANOBU IWANAGA**. *Appl. Sci.*, 2018, vol. 8, 1328
- **BONGSEOK CHOI et al.** *Chem. Commun.*, 2015, vol. 51, 11470-11473
- **MASANOBU IWANAGA et al.** *Nanoscale*, 2016, vol. 8, 11099-11107
- Frederich Wooten, Optical Properties of Solids. Academic Press, 1972, 52-65